# EUROPEAN PATENT SPECIFICATION

(11) **EP 2 512 381 B1**
(45) Date of publication and mention of the grant of the patent: **25.10.2017**
(21) Application number: 10838327.4
(22) Date of filing: 17.12.2010
(51) Int. Cl.: A61F 2/38, A61F 2/46

(54) **PATIENT-ADAPTED AND IMPROVED ORTHOPEDIC IMPLANTS, DESIGNS AND RELATED TOOLS**
PATIENTENADAPTIERTE UND VERBESSERTE ORTHOPÄDISCHE IMPLANTATE, ENTWÜRFE UND ZUGEHÖRIGE INSTRUMENTE
IMPLANTS ORTHOPÉDIQUES ADAPTÉS À UN PATIENT ET AMÉLIORÉS, MODÈLES ET OUTILS ASSOCIÉS

(30) Priority: 24.02.2010 US 712072; 25.02.2010 US 660529; 21.04.2010 US 799299; 22.04.2010 US 799355; 28.04.2010 US 799641; 18.12.2009 US 284458 P; 10.12.2010 US 965493; 23.06.2010 US 821301; 09.03.2010 US 339766 P
(43) Date of publication of application: 24.10.2012
(73) Proprietor: ConforMIS, Inc., Billerica, MA 01821 (US)
(72) Inventor: BOJARSKI, Raymond, A., Attleboro, MA 02703 (US); LANG, Philipp, Lexington, MA 02421 (US); CHAO, Nam, Marlborough, MA 01752 (US); FITZ, Wolfgang, Sherborn, MA 01770 (US); SLAMIN, John, Wrentham, MA 02093 (US); STEINES, Daniel, Lexington, MA 02421 (US); MINAS, Thomas, Dover, MA 02030 (US)
(74) Representative: Grund, Martin
(86) International application number: PCT/US2010/061141
(87) International publication number: WO 2011/075697

(56) References cited:
- EP-A1- 1 329 205
- WO-A1-2010/151564
- US-A- 5 171 244
- US-A- 5 968 099
- US-A1- 2006 009 853
- US-A1- 2006 190 086
- US-A1- 2007 239 165
- US-A1- 2008 058 945
- US-A1- 2008 058 945
- US-A1- 2008 119 938
- US-A1- 2009 228 113

## Description

### TECHNICAL FIELD

The invention relates to improved and/or patient-adapted (e.g., patient-specific and/or patient-engineered) orthopedic implants and tools, as well as related methods, design and models.

### BACKGROUND

Generally, a diseased, injured or defective joint, such as, for example, a joint exhibiting osteoarthritis, has been repaired using standard off-the-shelf implants and other surgical devices. Only recently has the concept of patient-specific implants tailored to an individual patient's joint become available. Such patient-specific implants, such as the iUni® and iDuo®, offer advantages over the traditional "several-size-fits-all" approach such as a better fit, more natural movement, reduction in the amount of bone removed during surgery and a less invasive procedure. Such patient-specific implants generally can be created from images of the patient's joint. Based on the images, the patient-specific implant can be created both to include surfaces that match existing surfaces in the joint, as well as to include surfaces that approximate an ideal and/or healthy surface that does not exist in the patient prior to any procedure. Implants also can be selected using imaging tests thereby optimizing the articular fit to select articular features or parameters.
US 2009/228,113 A1 describes a tibial tray configured to be fit into a tibial plateau of a patient.

### SUMMARY

The invention is defined in the claims.

### BRIEF DESCRIPTION OF THE DRAWINGS

The foregoing and other objects, aspects, features, and advantages of embodiments will become more apparent and may be better understood by referring to the following description, taken in conjunction with the accompanying drawings, in which:
FIGS. 1 and 2 are flow charts illustrating a process that includes selecting and/or designing a patient-adapted implant;
FIG. 3 is a flow chart illustrating a process for generating a model;
FIGS. 4 and 5 are flow charts showing exemplary steps taken in assessing a joint using a model;
FIG. 6 illustrates a set of models showing two perspectives of a patient's femur and two perspectives of a model of a patient's distal tibia;
FIG. 7 illustrates a set of models showing and image of a patient's distal femur with a patient-adapted guide tool, an image of a patient's distal femur with a patient-adapted unicompartmental femoral implant component, an image of a patient's proximal tibia with a patient-adapted guide tool and an image of a patient's proximal tibia with a patient-adapted unicompartmental tibial implant component;
FIG. 8 illustrates a set of models showing two perspectives of a patient's femur and two perspectives of a model of a patient's distal tibia;
FIG. 9 illustrates a set of models showing and image of a patient's distal femur with a patient-adapted guide tool, an image of a patient's distal femur with a patient-adapted bicompartmental femoral implant component, an image of a patient's proximal tibia with a patient-adapted guide tool and an image of a patient's proximal tibia with a patient-adapted bicompartmental tibial implant component;
FIG. 10 shows an example of a user interface from a computer software program for generating models;
FIG. 11 is a flow chart illustrating high level processes of an exemplar computer software program;
FIG. 12 is a flow chart illustrating the process of assessing and selecting and/or designing one or more implant component features and/or measurements;
FIG. 13 shows a coronal view of a patient's femoral bone and five standard resection cuts;
FIGS. 14A through 14F illustrate exemplary curvatures for one of more condylar coronal or sagittal curvatures; FIG. 14A corresponds to a section of a circle and shows two exemplary tangent radii of curvature; FIGS. 14B through 14F each correspond to a section of a non-circular shape and depict tangent radii of curvature having different lengths; FIG. 14F includes a straight line and no tangent radius of curvature;
FIG. 15 illustrates a design for a femoral implant;
FIG. 16 shows unicompartmental medial and lateral tibial implant components without a polyethylene layer;
FIG. 17 shows unicompartmental medial and lateral tibial implant components with a polyethylene layer;
FIG. 18 depicts exemplary tibial cuts for the medial and lateral plateaus and corresponding femoral cuts;
FIG. 19 depicts a tilted tibial surface to correspond to a tilted sagittal curve of the femoral component;
FIGS. 20 and 21 depict exemplary cross-sections of tibial implant components having a post projecting from the bone facing surface of the implant component;
FIG. 22 depicts two views of an intercondylar housing on a cruciate-retaining femoral implant;
FIGS. 23A-23F show exemplary spacers or trials or inserts; specifically, FIG. 23A depicts a top view of a medial balancer chip; FIG. 23B depicts a side view of a set of four medial balancer chips; FIG. 23C depicts a medial balancing chip being inserted between a femur and tibia; FIG. 23D depicts the medial balancing chip insert in place between a femur and tibia; FIG. 23E shows a cutting guide to be attached to the medial balancing chip insert; FIG. 23F shows the medial balancing chip insert used as a cut guide for the tibia;
FIG. 24 depicts a set of three medial spacer block inserts;
FIG. 25 depicts a set of two medial femoral trials;
FIG. 26 depicts a medial spacer block and a medial femoral trial in place;
FIG. 27 depicts a set of three medial tibial component insert trials;
FIG. 28A, 28B and 28C depict the process of placing and adding various tibial component insert trials.

### DETAILED DESCRIPTION

### 1. Introduction

When a surgeon uses a traditional off-the-shelf implant to replace a patient's joint, for example, a knee joint, hip joint, or shoulder joint, certain features of the implant typically do not match the particular patient's biological features. These mismatches can cause various complications during and after surgery. For example, surgeons may need to extend the surgery time and apply estimates and rules of thumb during surgery to address the mismatches. For the patient, complications associated with these mismatches can include pain, discomfort, soft tissue impingement, and an unnatural feeling of the joint during motion, e.g., so-called mid-flexion instability, as well as an altered range of movement and an increased likelihood of implant failure. In order to even fit a traditional implant component to a patient's articular bone, surgeons typically remove substantially more of the patient's bone than otherwise would be needed, e.g., to clear diseased bone from the site. This removal of substantial portions of the patient's bone frequently diminishes the patient's bone stock to the point that only one subsequent revision implant is possible.

Certain embodiments of the implants, guide tools, and related methods of designing, making, and using the implants and guide tools described herein can be applied to any joint including, without limitation, a spine, spinal articulations, an intervertebral disk, a facet joint, a shoulder joint, an elbow, a wrist, a hand, a finger joint, a hip, a knee, an ankle, a foot, or a toe joint. Furthermore, various embodiments described herein can apply to methods and procedures, and the design of methods and procedures, for implanting the implants described herein and/or using the guide tools described herein.

### 1.1 Patient-adapted features

Certain embodiments relate to patient-adapted implants, guide tools, and related methods. Patient-adapted features of an implant component, guide tool or related implantation method can be achieved by analyzing imaging test data and selecting and/or designing (e.g., preoperatively selecting from a library and/or preoperatively designing) an implant component, a guide tool, and/or a procedure having a feature that is matched and/or optimized for the particular patient's biology. Accordingly, the patient-adapted implant components, guide tools, and/or methods include one or more patient-adapted features. Patient-adapted features can be patient-specific or patient-engineered.

Some embodiments relate to articular implant components having one or more patient-specific features adapted to match one or more of the patient's biological features, such as one or more biological/anatomical structures, alignments, kinematics, and/or soft tissue impingements. Accordingly, the one or more patient-specific features of an implant component can include, but are not limited to, one or more implant component surfaces, such as surface contours, angles or bone cuts, and dimensions such as thickness, width, depth, or length of one or more aspects of the implant component. The patient-specific feature(s) of an implant component can be designed based on patient-specific data to substantially match one or more of the patient's biological features. In various embodiments described herein, the act of designing an implant component can include manufacturing the implant component having the related design features. For example, designing an implant component can include preoperatively establishing design features of the implant component and manufacturing the corresponding implant component.

Alternatively, patient-specific feature(s) of an implant component or guide tool can be achieved by analyzing imaging test data and selecting (e.g., preoperatively selecting from a library of implant components) the implant component that best fits one or more predetermined patient specific parameters that are derived from the imaging test.

Moreover, an implant component or guide tool can include a patient-specific feature that is both selected and designed. For example, an implant component initially can be selected (e.g., preoperatively selected from a library of implants) to have a feature with a larger or smaller dimension than the predetermined patient-specific dimension. Then, the implant component can be machined (if selected from an actual library of implant components) or manufactured (if selected from a virtual library of implant components) so that the larger-dimensioned or smaller-dimensioned implant feature is altered to have the patient-specific dimension.

In addition or alternatively, certain embodiments relate to patient-engineered implants, guide tools, and related methods. Some embodiments relate to articular implant components having one or more patient-engineered features optimized from patient-specific data to meet one or more parameters to enhance one or more of the patient's biological features, such as one or more biological/anatomical structures, alignments, kinematics, and/or soft tissue impingements. Accordingly, the one or more patient-engineered features of an implant component can include, but are not limited to, one or more implant component surfaces, such as surface contours, angles or bone cuts, and dimensions such as thickness, width, depth, or length of one or more aspects of the implant component. The patient-engineered feature(s) of an implant component can be designed and manufactured (e.g., preoperatively designed and manufactured) based on patient-specific data to substantially enhance or improve one or more of the patient's biological features.

As with the patient-specific feature(s) of an implant component or guide tool, the patient-engineered features of an implant component or guide tool can be designed and manufactured (e.g., preoperatively designed and manufactured) or they can be selected, for example, by selecting an implant component that best meets the one or more predetermined parameters that enhance one or more features of the patient's biology.

Moreover, an implant component or guide tool can include a patient-engineered feature that is both selected and designed. For example, an implant component initially can be selected (e.g., preoperatively selected from a library of implants) to have a feature with a larger or smaller dimension than the desired patient-engineered dimension. Then, the implant component can be machined (if selected from an actual library of implant components) or manufactured (if selected from a virtual library of implant components) so that the larger-dimensioned or smaller-dimensioned implant feature is altered to have the desired patient-engineered dimension.

### 1.2 Combinations of patient-adapted and standard features

In certain aspects, an implant, guide tool, and/or related method can include one or more patient-adapted features and one or more standard features. For example, the joint-facing surface of an implant component can include a patient-specific feature (e.g., curvature) in the axis or axes of motion and one or more standard features in other axes. Using a curvature in the direction of motion that is matched to the patient's curvature (patient-specific) or that is optimized based on the patient's curvature (patient-engineered) can help to maintain and/or improve the biomechanical aspects of the patient's joint.

In certain embodiments, implant components and/or related methods described herein can include a combination of patient-specific and patient-engineered features. For example, patient-specific data collected preoperatively can be used to engineer one or more optimized surgical cuts to the patient's bone and to design or select a corresponding implant component having or more bone-facing surfaces or facets (i.e., "bone cuts") that specifically match one or more of the patient's resected bone surfaces. The surgical cuts to the patient's bone can be optimized (i.e., patient-engineered) to enhance one or more parameters, such as: (1) deformity correction and limb alignment (2) maximizing preservation of bone, cartilage, or ligaments, (3) maximizing preservation and/or optimization of other features of the patient's anatomy, such as trochlea and trochlear shape, (4) restoration and/or optimization of joint kinematics or biomechanics, and/or (5) restoration or optimization of joint-line location and/or joint gap width. Based on the optimized surgical cuts and, optionally, on other desired features of the implant component, the implant component's bone-facing surface can be designed or selected to, at least in part, negatively-match the shape of the patient's resected bone surface.

Optionally, the implant component can include other patient-adapted features. For example, the joint-facing surface can be designed to, at least in part, substantially negatively-match an opposing surface at the joint cavity (e.g., the surface of the patient's biological structure, native or resected, or the surface of an opposing implant component. In certain embodiments, the joint-facing surface of the implant component can include patient-adapted features such as a patient-specific sagittal curvature and a patient-engineered coronal curvature, or a patient-specific sagittal curvature and a patient-specific coronal curvature. These curvatures can be patient-specific in at least a portion or the entire joint-facing surface of the implant. Alternatively or in addition, one or more of these curvatures can be patient-adapted in one condyle, but not patient adapted in the other condyle. Moreover, the shape (e.g., curvature) of a first condyle can be used, for example, to generate a shape (e.g., curvature) for the second condyle.

### 1.4 Improved implants, guide tools and related methods

Certain embodiments are directed to implants, guide tools, and/or related methods that can be used to provide to a patient a pre-primary procedure and/or a pre-primary implant such that a subsequent, replacement implant can be performed with a second (and, optionally, a third, and optionally, a fourth) patient-adapted pre-primary implant or with a traditional primary implant. In certain embodiments, the pre-primary implant procedure can include 3, 4, 5, 6, 7, or more resection or surgical cuts to the patient's bone and the pre-primary implant can include on its corresponding bone-facing surface a matching number and orientation of bone-cut facets or surfaces.

In one illustrative embodiment, a first pre-primary joint-replacement procedure includes a patient-adapted implant component, guide tool, and/or related method. The patient-adapted implant component, guide tool, and/or related method can be preoperatively selected and/or designed from patient-specific data, such as one or more images of the patient's joint, to include one or more features that are patient-specific or patient-engineered. The features (e.g., dimensions, shape, surface contours) of the first pre-primary implant and, optionally, patient-specific data (e.g., features of the patient's resected bone surfaces and features of the patient's contralateral joint) can be stored in a database. When the first pre-primary implant fails, for example, due to bone loss or osteolysis or aseptic loosening at a later point in time (e.g., 15 years after the original implantation) a second implant can be implanted. For the second implant procedure, the amount of diseased bone can be assessed. If the amount of diseased bone to be resected is minimal, the patient-specific data can be used to select and/or design a second pre-primary procedure and/or a pre-primary implant. If the amount of diseased bone to be resected is substantial, a traditional primary procedure and a traditional implant can be employed.

Alternatively, certain embodiments are directed to implants, guide tools, and/or related methods that can be used to provide to a patient a primary procedure and/or a primary implant such that a subsequent replacement implant can be used as part of a traditional revision procedure. Certain embodiments are directed to implants, guide tools, and/or related methods that can be used to provide a patient-adapted revision implant. For example, following a traditional implant, a subsequent revision can include a patient-adapted procedure and/or a patient-adapted implant component as described herein.

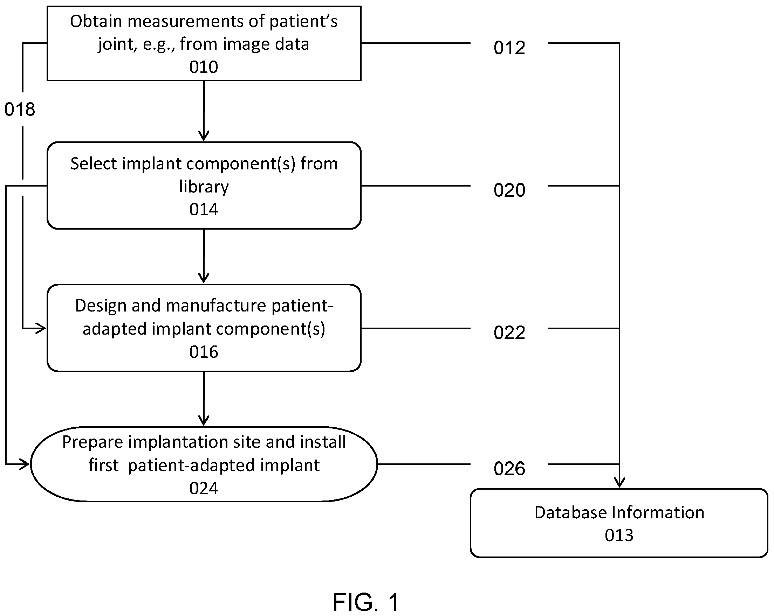

**FIG. 1** is a flow chart illustrating a process that includes selecting and/or designing a first patient-adapted implant, for example, a pre-primary implant. First, using the techniques described herein or those suitable and known in the art, measurements of the target joint are obtained **010.** This step can be repeated multiple times, as desired. Optionally, a virtual model of the joint can be generated, for example, to determine proper joint alignment and the corresponding resection cuts and implant component features based on the determined proper alignment. This information can be collected and stored **012** in a database **013.** Once measurements of the target joint are obtained and analyzed to determine resection cuts and patient-adapted implant features, the patient-adapted implant components can be selected **014** (e.g., selected from a virtual library and subsequently manufactured, or selected from a physical library of implant components). Alternatively, or in addition, one or more implant components with best-fitting and/or optimized features can be selected **014** (e.g., from a library) and then further designed and manufactured **016.** Alternatively or in addition, one or more implant components with best-fitting and/or optimized features can be designed and manufactured **018** without an initial selection from a library. Using a virtual model to assess the selected or designed implant component(s), this process also can be repeated as desired (e.g., before one or more physical components are selected and/or generated). The information regarding the selected and/or designed implant component(s) can be collected and stored **020, 022** in a database **013.** Once a desired first patient-adapted implant component or set of implant components is obtained, a surgeon can prepare the implantation site and install the first implant **024.** The information regarding preparation of the implantation site and implant installation can be collected and stored **026** in a database **013.** In this way, the information associated with the first pre-primary implant component is available for use by a surgeon for subsequent implantation of a second pre-primary or a primary implant.

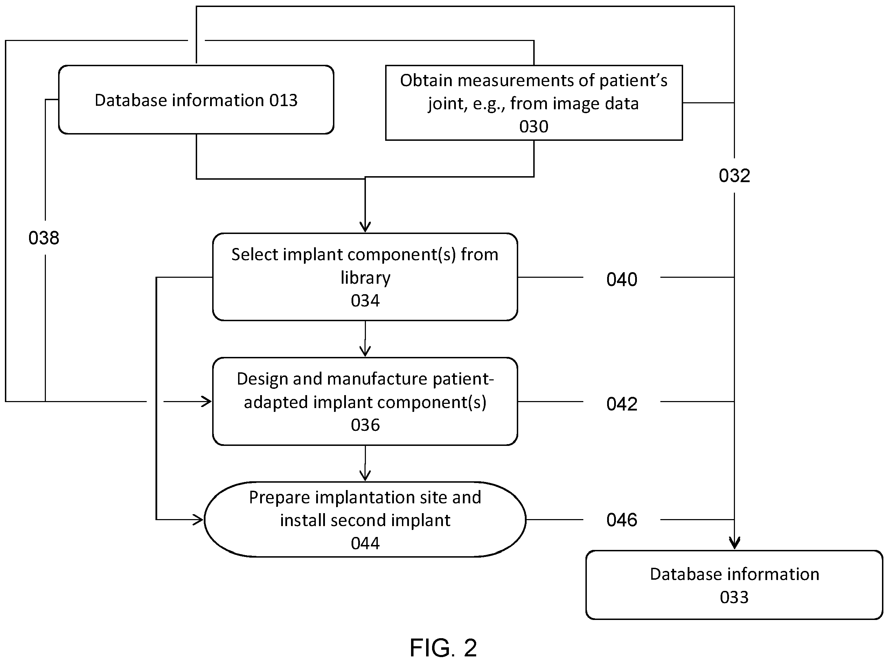

**FIG. 2** is a flow chart illustrating a process that includes selecting and/or designing a second implant. In certain embodiments, the second implant can be a traditional primary implant. Alternatively, the second implant can be a patient-adapted implant, which optionally can be used as second pre-primary implant that allows for a subsequent (i.e., third) primary implant using a traditional implant.

### 2. Exemplary implant systems and patient-adapted features

The concepts described herein can be embodied in various types of implants including, but not limited to, knee-joint implants, hip-joint implants, shoulder-joint implants, and spinal implants, as well as related guide tools and methods.

In certain embodiments described herein, an implant or implant system can include one, two, three, four or more components having one or more patient-adapted features that substantially match one or more of the patient's biological features, for example, a dimension or measurement of an anatomical/biological structure, such as bone, cartilage, tendon, or muscle; a distance or space between two aspects or between two different biological structures; and a biomechanical or kinematic quality or measurement of the patient's biology. In addition or alternatively, an implant component can include one or more features that are engineered to optimize or enhance one or more of the patient's biological features, for example, (1) deformity correction and limb alignment (2) maximizing preservation of bone, cartilage, or ligaments, (3) maximizing preservation and/or optimization of other features of the patient's anatomy, such as trochlea and trochlear shape, (4) restoration and/or optimization of joint kinematics or biomechanics, and/or (5) restoration or optimization of joint-line location and/or joint gap width. In addition, an implant component can be designed and/or manufactured to include one or more standard (i.e., non-patient adapted) features. Each feature of an implant can be patient-specific, patient-engineered, or standard. For a knee implant, thirteen exemplary features can include: (1) femoral implant component M-L dimension, (2) femoral implant component A-P dimension, (3) femoral implant component bone cut, (4) femoral implant component sagittal curvature, (5) femoral implant component coronal curvature, (6) femoral implant component inter-condylar distance, (7) femoral implant component notch location / geometry, (8) tibial implant component M-L dimension, (9) tibial implant component A-P dimension, (10) tibial implant component insert inter-condylar distance, (11) tibial implant component insert lock, (12) tibial implant component metal backing lock, and (13) tibial implant component metal backing perimeter.

### 2.2 Joint-facing surface of an implant component

In certain embodiments, the joint-facing surface of an implant component can be designed to positively-match one or more features of the patient's existing cartilage surface and/or healthy cartilage surface and/or a calculated cartilage surface, or to positively-match one or more features of the patient's existing subchondral bone surface and/or healthy subchondral bone surface and/or a calculated subchondral bone surface. Alternatively, it can substantially-negatively match one or more features of the patient's existing cartilage surface and/or healthy cartilage surface and/or a calculated cartilage surface, or it can substantially-negatively match one or more features of the patient's existing subchondral bone surface and/or healthy subchondral bone surface and/or a calculated subchondral bone surface on the opposing articular surface in the joint. Corrections can be performed to the shape of diseased cartilage or subchondral bone to re-establish a normal or near normal cartilage or articular shape that can then be incorporated into the shape of the joint-facing surface of the component. These corrections can be implemented and, optionally, tested in virtual two-dimensional and three-dimensional models.

Certain embodiments include, in addition to a first implant component, a second implant component having an opposing joint-facing surface. However, when the first implant component's joint-facing surface includes a feature designed to match a patient's biological feature, a second implant component having a feature designed to match that feature of the first implant component also matches the patient's same biological feature. By way of illustration, when a joint-facing surface of a first component is designed to positively-match at least a portion of the patient's cartilage shape, the opposing joint-facing surface of the second component designed to match that feature of the first implant component also negatively-matches the same portion of the patient's cartilage shape. When the joint-facing surface of the first component is designed to positively-match at least a portion of a patient's subchondral bone shape, the opposing joint-facing surface of the second component designed to match that feature of the first implant component also negatively-matches the same portion of the patient's subchondral bone shape. When the joint-facing surface of the first component is designed to positively-match at least a portion of a patient's cortical bone, the joint-facing surface of the second component designed to match that feature of the first implant component also negatively-matches the same portion of the patient's cortical bone shape. When the joint-facing surface of the first component is designed to positively-match at least a portion of a patient's endosteal bone shape, the opposing joint-facing surface of the second component designed to match that feature of the first implant component also negatively-matches the same portion of the patient's endosteal bone shape. When the joint-facing surface of the first component is designed to positively-match at least a portion of a patient's bone marrow shape, the opposing joint-facing surface of the second component designed to match that feature of the first implant component also negatively-matches the same portion of the patient's bone marrow shape.

### 3. Measuring biological features

Data for obtaining measurements of a patient' joint, for example, relative-position measurements, length or distance measurements, curvature measurements, surface contour measurements, thickness measurements (in one location or across a surface), volume measurements (filled or empty volume), density measurements, and other measurements, can be obtained using any suitable technique. For example, one dimensional, two-dimensional, and/or three-dimensional measurements can be obtained using data collected from mechanical means, laser devices, electromagnetic or optical tracking systems, molds, materials applied to the articular surface that harden as a negative match of the surface contour, and/or one or more imaging techniques described above and/or known in the art. Data and measurements can be obtained non-invasively and/or preoperatively. Alternatively, measurements can be obtained intraoperatively, for example, using a probe or other surgical device during surgery.

In certain embodiments, imaging data collected from the patient, for example, imaging data from one or more of x-ray imaging, digital tomosynthesis, cone beam CT, non-spiral or spiral CT, non-isotropic or isotropic MRI, SPECT, PET, ultrasound, laser imaging, photo-acoustic imaging, is used to qualitatively and/or quantitatively measure one or more of a patient's biological features, one or more of normal cartilage, diseased cartilage, a cartilage defect, an area of denuded cartilage, subchondral bone, cortical bone, endosteal bone, bone marrow, a ligament, a ligament attachment or origin, menisci, labrum, a joint capsule, articular structures, and/or voids or spaces between or within any of these structures. The qualitatively and/or quantitatively measured biological features can include, but are not limited to, one or more of length, width, height, depth and/or thickness; curvature, for example, curvature in two dimensions (e.g., curvature in or projected onto a plane), curvature in three dimensions, and/or a radius or tangent radii or curvature; shape, for example, two-dimensional shape or three-dimensional shape; area, for example, surface area and/or surface contour; perimeter shape; and/or volume of, for example, the patient's cartilage, bone (subchondral bone, cortical bone, endosteal bone, and/or other bone), ligament, and/or voids or spaces between them.

In certain embodiments, measurements of biological features can include any one or more of the illustrative measurements identified in **Table 2.**

**Table 2: Exemplary patient-specific measurements of anatomical features that can be used in the selection and/or design of an implant**

| **Anatomical feature** | **Exemplary measurement** |
|---|---|
| **Joint-line, joint gap** | - Location relative to proximal reference point |
| | - Location relative to distal reference point |
| | - Angle |
| | - Gap distance between opposing surfaces in one or more locations |
| | - Location, angle, and/or distance relative to contralateral joint |
| **Soft tissue tension and/or balance** | - Joint gap distance |
| | - Joint gap differential, e.g., medial to lateral |
| **Medullary cavity** | - Shape in one or more dimensions |
| | - Shape in one or more locations |
| | - Diameter of cavity |
| | - Volume of cavity |
| **Subchondral bone, Cortical bone, Endosteal bone, Cartilage** | - Shape in one or more dimensions |
| | - Shape in one or more locations |
| | - Thickness in one or more dimensions |
| | - Thickness in one or more locations |
| | - Angle, e.g., resection cut angle |
| **Intercondylar notch** | - Shape in one or more dimensions |
| | - Location |
| | - Height in one or more locations |
| | - Width in one or more locations |
| | - Depth in one or more locations |
| | - Angle, e.g., resection cut angle |
| **Medial condyle, Lateral condyle, Trochlea, Medial trochlea, Central trochlea, Lateral trochlea, Entire tibia, Medial tibia, Lateral tibia, Entire patella, Medial patella, Central patella, Lateral patella** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |
| **Femoral head, Acetabulum, Gelnoid, Humeral Head, Ankle joint**, **Elbow, Wrist, Hand, Finger, Spine, Spinal facet joint** | - 2D and/or 3D shape of a portion or all |
| | - Height in one or more locations |
| | - Length in one or more locations |
| | - Width in one or more locations |
| | - Depth in one or more locations |
| | - Thickness in one or more locations |
| | - Curvature in one or more locations |
| | - Slope in one or more locations and/or directions |
| | - Angle, e.g., resection cut angle |

Depending on the clinical application, a single or any combination of all of the measurements described in **Table 2** and/or known in the art can be used. Additional patient-specific measurements and information that be used in the evaluation can include, for example, patient age, weight, gender, ethnicity, activity level, and overall health status.

The patient-specific measurements selected for the evaluation then can be used to select (e.g., from a library) and/or design an implant component having one or more measurements corresponding to or derived from the one or more of the assessed patient-specific measurements. For example, the implant component can include one or more patient-specific measurements and/or one or more patient-engineered measurements.

### 3.1 Generating a model

In certain embodiments, a practitioner can generate a model of a patient's joint. Specifically, a practitioner can use the patient-specific data and/or measurements described above to generate a model that includes one or more biological features of the patient (e.g., biological structure, such as bone, cartilage, and or tissue at or relating to a joint) and, optionally, one or more patient-engineered resection cuts, patient-adapted guide tools, and/or patient-adapted implant components. In certain embodiments, a model that includes the patient's biological feature(s) can be used to directly generate a patient-engineered resection cut strategy, a patient-adapted guide tool design, and/or a patient-adapted implant component design for a surgical procedure (e.g., without the model itself including the resection cuts, the guide tool, and/or the implant component). In other embodiments, the model that includes the patient's biological feature(s) also can include or show, as part of the model, one or more resection cuts, guide tools, and/or the implant components that have been designed specifically for patient. Moreover, resection cuts, guide tools, and/or implant components can be modeled separate from a model of a particular patient's biological feature.

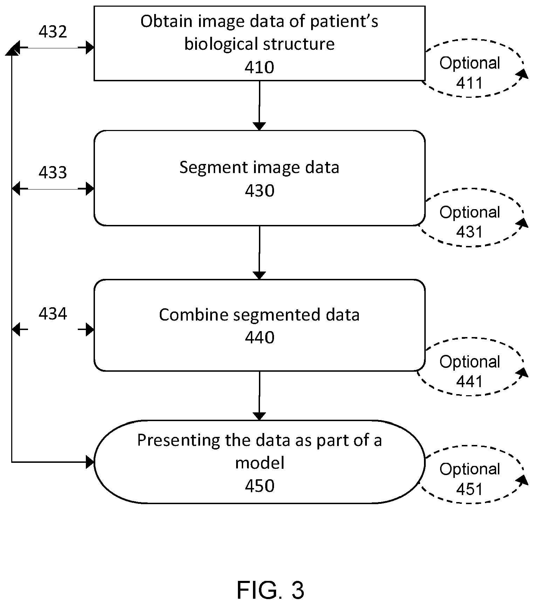

Various methods can be used to generate a model. As illustrated in **FIG. 3****,** in certain embodiments the method of generating a model of a patient's biological feature (and/or a patient-specific feature of a guide tool or implant component) can include one or more of the steps of obtaining image data of a patient's biological structure **410;** segmenting image data **430;** combining segmented data **440;** and presenting the data as part of a model **450.**

Image data can be obtained **410** from near or within the patient's biological structure of interest. For example, pixel or voxel data from one or more radiographic images of a patient's joint can be obtained. In this or a subsequent step, one or more pixels or voxels can be converted into one or a set of values. For example, a single pixel/voxel or a group of pixel/voxels can be converted to coordinate values, e.g., a point in a 2D or 3D coordinate system. The set of values also can include a value corresponding to the pixel/voxel intensity or relative grayscale color. Moreover, the set of values can include information about neighboring pixels or voxels, for example, information corresponding to relative intensity or grayscale color and or information corresponding to relative position.

Then, the image data can be segmented **430** to identify those data corresponding to a particular biological feature of interest. Optionally, the segmented data can be combined. For example, in a single image segmented and selected data points (e.g., derived from pixels or voxels) and/or other data can be combined to create a line representing the surface outline of a biological structure. Moreover, the segmented and selected data from multiple images can be combined to create a 3D representation of the biological structure.

Optionally, the 3D representation of the biological structure can be manipulated, smoothed or corrected, for example, by employing a 3D parametric surface, for example, a polygon or non-uniform rational B-spline (NURBS). For a description of various parametric surface representations see, for example Foley, J. D. et al., Computer Graphics: Principles and Practice in C; Addison-Wesley, 2nd edition, 1995). Various methods are available for creating a parametric surface. For example, the 3D representation can be converted directly into a parametric surface, for example, by connecting data points to create a surface of polygons and applying rules for polygon curvatures, surface curvatures, and other features. Alternatively, a parametric surface can be best-fit to the 3D representation, for example, using publicly available software such as Geomagic® software (Research Triangle Park, N.C.).

Then, the data can be presented as part of a model **450,** for example, a patient-specific virtual model that includes the biological feature of interest. Optionally, the data associated with one or more biological features can be transferred to one or more resection cuts, guide tools, and/or implant components, which also can be included as part of the same model or in a different model. The virtual model(s) can be used to generate one or more patient-adapted guide tools and/or implant components for surgical use, for example, using CAD software and one or more of the several manufacturing techniques described below.

As will be appreciated by those of skill in the art, one or more of these steps **410, 430, 440, 450** can be repeated **411, 431, 441, 451** as often as desired to achieve the desired result. Moreover, the steps can be repeated reiteratively **432, 433, 434.** As will be appreciated by those of skill in the art, the practitioner can proceed directly **433** from the step of segmenting image data **430** to presenting the data as part of a model **450**. Data, models and/or any related guide tools or implant components can be collected in one or more libraries for subsequent use for the same patient or for a different patient (e.g., a different patient with similar data).

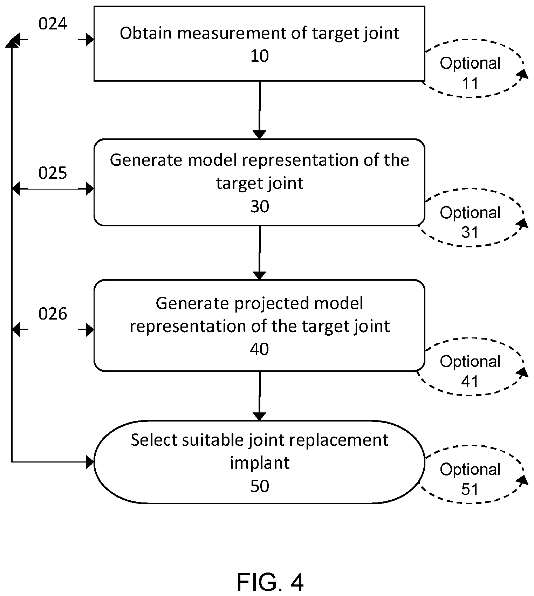

**FIG. 4** is a flow chart showing exemplary steps taken by a practitioner in assessing a joint using a model. First, a practitioner obtains a measurement of a target joint **10.** The step of obtaining a measurement can be accomplished, for example, based on an image of the joint. This step can be repeated as necessary to obtain a plurality of measurements, for example, from one or more images of the patient's joint, in order to further refine the joint assessment process. Once the practitioner has obtained the necessary measurements, the information can be used to generate a model representation of the target joint being assessed **30.** This model representation can be in the form of a topographical map or image. The model representation of the joint can be in one, two, or three dimensions. It can include a virtual model and/or a physical model. More than one model can be created, if desired. Either the original model, or a subsequently created model, or both can be used.

After the model representation of the joint is generated **30,** the practitioner optionally can generate a projected model representation of the target joint in a corrected condition **40,** e.g., based on a previous image of the patient's joint when it was healthy, based on an image of the patient's contralateral healthy joint, based on a projected image of a surface that negatively matches the opposing surface, or a combination thereof. This step can be repeated, as necessary or as desired. Using the difference between the topographical condition of the joint and the projected image of the joint, the practitioner can then select a joint implant **50** that is suitable to achieve the corrected joint anatomy. As will be appreciated by those of skill in the art, the selection process **50** can be repeated **51** as often as desired to achieve the desired result. Additionally, it is contemplated that a practitioner can obtain a measurement of a target joint **10** by obtaining, for example, an x-ray, and then selects a suitable joint replacement implant **50.**

As will be appreciated by those of skill in the art, the practitioner can proceed directly from the step of generating a model representation of the target joint **30** to the step of selecting a suitable joint replacement implant **50** as shown by the arrow **32.** Additionally, following selection of suitable joint replacement implant **50,** the steps of obtaining measurement of target joint **10,** generating model representation of target joint **30** and generating projected model **40,** can be repeated in series or parallel as shown by the flow **24, 25,26.**

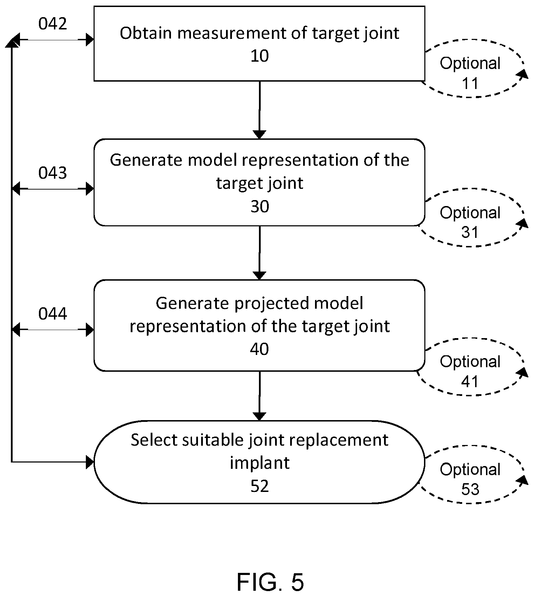

**FIG. 5** is an alternate flow chart showing steps taken by a practitioner in assessing a joint. First, a practitioner obtains a measurement of a target joint **10.** The step of obtaining a measurement can be accomplished by taking an image of the joint. This step can be repeated, as necessary **11** to obtain a plurality of images in order to further refine the joint assessment process. Once the practitioner has obtained the necessary measurements, the information is used to generate a model representation of the target joint being assessed **30.** This model representation can be in the form of a topographical map or image. The model representation of the joint can be in one, two, or three dimensions. The process can be repeated **31** as necessary or desired. It can include a physical model. After the model representation of the joint is assessed **30,** the practitioner optionally can generate a projected model representation of the target joint in a corrected condition **40.** This step can be repeated **41** as necessary or desired. Using the difference between the topographical condition of the joint and the projected image of the joint, the practitioner can then design a joint implant **52** that is suitable to achieve the corrected joint anatomy, repeating the design process **53** as often as necessary to achieve the desired implant design. The practitioner can also assess whether providing additional features, such as rails, keels, lips, pegs, cruciate stems, or anchors, crossbars, etc. will enhance the implant's performance in the target joint.

As will be appreciated by those of skill in the art, the practitioner can proceed directly from the step of generating a model representation of the target joint **30** to the step of designing a suitable joint replacement implant **52** as shown by the arrow **38.** Similar to the flow shown above, following the design of a suitable joint replacement implant **52,** the steps of obtaining measurement of target joint **10,** generating model representation of target joint **30** and generating projected model **40**, can be repeated in series or parallel as shown by the flow **42, 43, 44.**

### 3.2 Variations to biological surfaces at the joint

In certain embodiments, the reference points and/or measurements described above can be processed using mathematical functions to derive virtual, corrected surfaces, which may represent an ideal or desired surface from which a patient-specific implant can be designed. For example, one or more surfaces or dimensions of a biological structure can be modeled, altered, added to, changed, deformed, eliminated, corrected and/or otherwise manipulated (collectively referred to herein as "variation" of an existing surface or structure within the joint).

In addition to osteophytes and subchondral voids, the methods, designs, resection cut strategies, guide tools, and implant components described herein can be used to address various other patient-specific joint defects or phenomena. In certain embodiments, correction can include the virtual removal of diseased or damaged tissue (e.g., cartilage, bone, or other types of tissue), for example, osteochondritic tissue, necrotic tissue, torn tissue. In certain embodiments, correction can include the virtual removal of subchondral cysts. The bone-facing surface of the implant is then derived after the subchondral cyst has been virtually removed.

In one embodiment, a correction can include the virtual removal of subchondral cysts. The bone-facing surface of the implant is then derived after the subchondral cyst has been virtually removed.

In another embodiment, a correction can include the virtual removal of articular defects. The bone facing surface of the implant is then derived after the articular defect has been virtually removed. In this embodiment, the defect may then be filled intraoperatively with bone cement, bone graft or other bone fillers. Alternatively, the articular defect can be integrated in the shape of the bone facing surface of the implant.

In certain embodiments, models can be generated to show defects of interest in a patient's joint. For example, a model or set of models of a patient's joint can be generated showing defects of interest and, optionally, another model or set of models can be generated showing no defects (e.g., as defect and reference, or before and after models). Alternatively, or in addition, the same or additional models can be generated with and/or without resection cuts, guide tools, and/or implant components positioned in the model. Moreover, the same or additional models can be generated to show defects of interest that interfere with one or more resection cuts, guide tools, and/or implant components. Such models, showing defects of interest, resection cuts, guide tools, implant components, and/or interfering defects of interest, can be particularly useful as a reference or guide to a surgeon or clinician prior to and/or during surgery, for example, in identifying proper placement of a guide tool or implant component at one or more steps in a surgery, and/or in identifying features of a patient's anatomy that he or she may want to alter during one or more steps in a surgery. Accordingly, such models that provide, for example, patient-specific renderings of implant assemblies and defects of interest (e.g., osteophyte structures) together with bone models, are useful in aiding surgeons and clinicians in surgery planning and/or during surgery.

In certain embodiments, a model or set of models of a patient's biological structure are obtained and/or generated to show one or more defects of interest, one or more resection cuts, one or more guide tools, one or more implant components, and/or a combination of any of these. For models that include defects of interest, the defects can be categorized and differentiated into various groups and displayed on the model or set of models in any way that conveys the appropriate information to a surgeon or clinician. For example, two or more defects can be differentiated into categories based on defect type, defect location, severity of the defect, potential interference with a guide tool or implant component, and/or any one or more other useful categories.

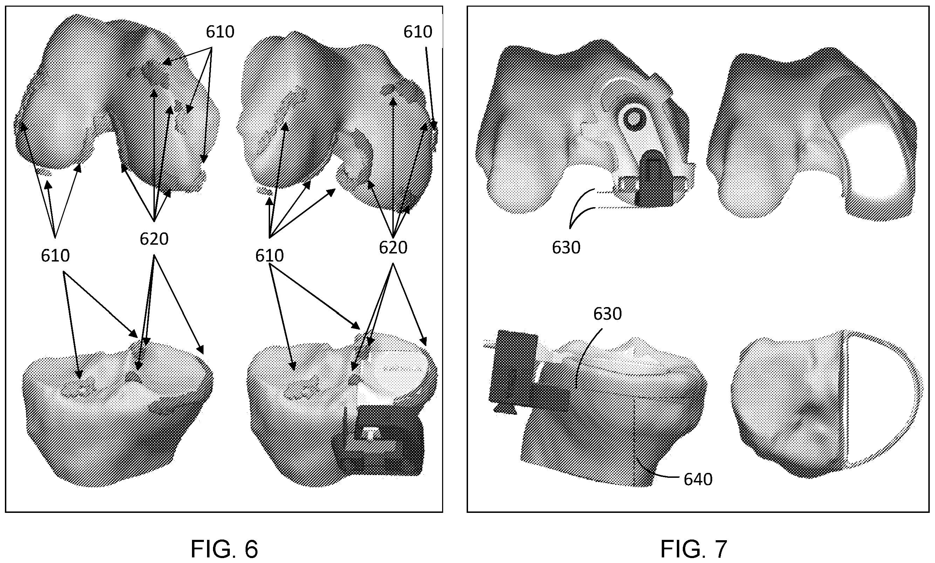

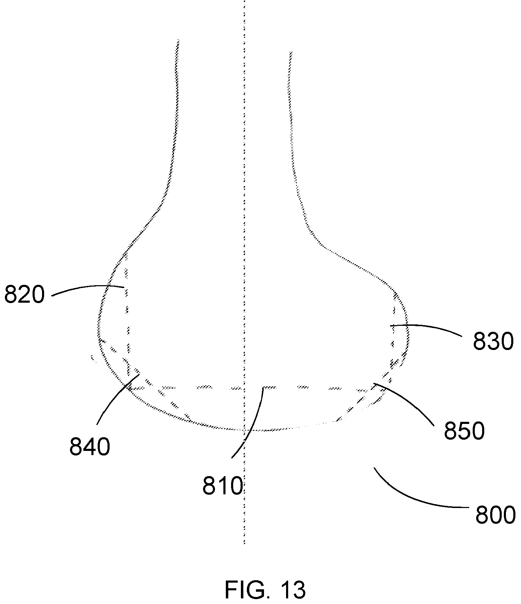

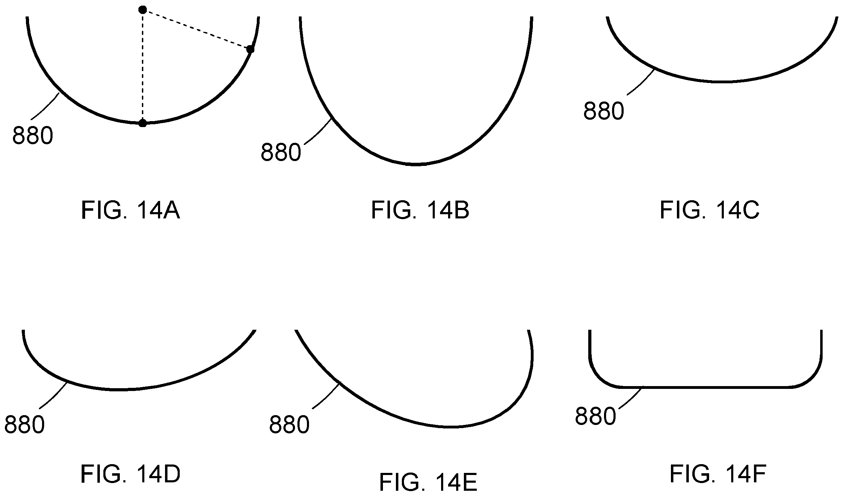

In certain embodiments, one or more additional models or set of models of the patient's biological structure also can be generated and conveyed to the surgeon or clinician to show an additional presence, or absence, of one or more defects of interest, one or more resection cuts, one or more guide tools, and/or one or more implant components, or any combination of these. **FIGS. 13** and **14** illustrate models for one particular patient receiving a single compartment knee implant having both femoral and tibial implant components. In a first set of models, illustrated in **FIG. 6****,** the top panel shows two perspectives of a patient's distal femur and the bottom panel shows two images of the patient's proximal tibia, without (left image) and with (right image) a patient-adapted guide tool. In both the top and bottom panels, defects, osteophytes in this example, are categorized and differentiated into two colors, beige and red. The beige-colored osteophytes **610** depict osteophytes that do not interfere with placement of the guide tools or implant components. The red-colored osteophytes **620** depict osteophytes that will interfere with placement of a guide tool or an implant component. Accordingly, the categorization of the osteophytes serve as a guide or reference to the surgeon or clinician in determining which osteophytes should be removed prior to placement of the guide tools or implant components.

In a second set of models, illustrated in **FIG. 7****,** the top panel shows two images of a patient's distal femur with a patient-adapted guide tool (left image) and with a patient-adapted unicompartmental femoral implant component (right image). The bottom panel shows two images of the patient's proximal tibia, with a patient-adapted guide tool (left image) and with a patient-adapted unicompartmental tibial implant component (right image). The images in this figure show no osteophytes. Instead, these images show how each guide tool and implant component engages the patient's biological structure. In addition, the images showing the guide tools also show corresponding resection planes **630** and the patient's tibial mechanical axis **640.** Accordingly, these images can serve as a guide or reference to the surgeon or clinician in planning and/or determining the surgical placement of the guide tools, implant components, and related resection cuts.

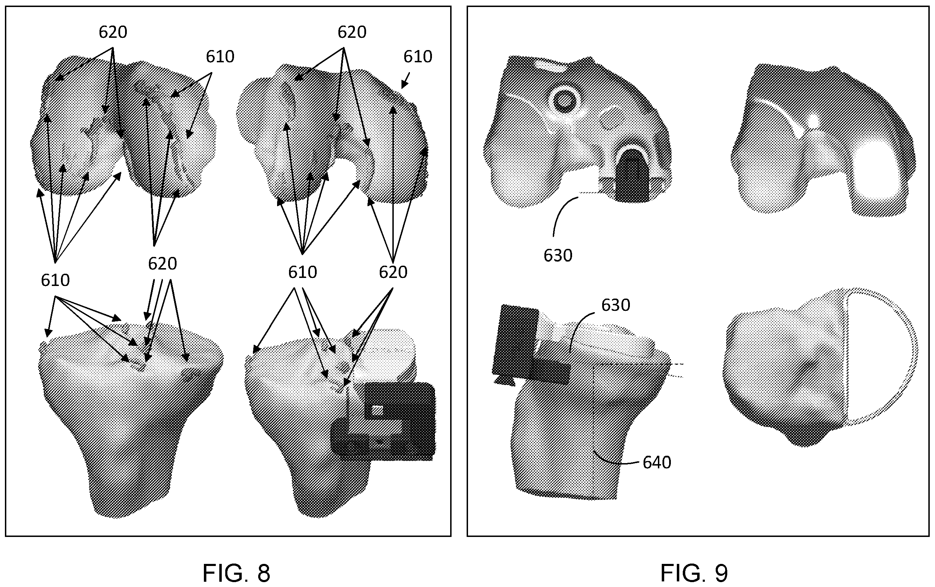

**FIGS. 8** and 9 illustrate models for a different patient receiving a bicompartmental knee implant having both femoral and tibial implant components. The features in **FIGS. 8 and 9** are similar to those described above for **FIGS. 6** and **7****.** In comparing the models for the two different individuals, it is clear that the individual receiving the unicompartmental knee implant **(****FIGS. 6** and **7****)** has substantially more osteophyte coverage than the individual receiving the bicompartmental knee implant **(****FIGS. 8** and **9****).**

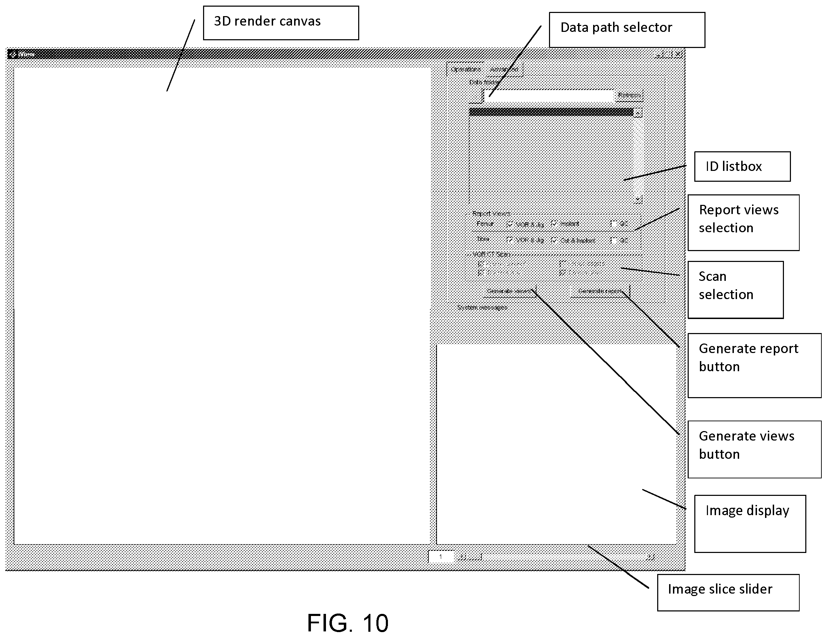

Computer software programs to generate models of patient-specific renderings of implant assembly and defects (e.g., osteophyte structures), together with bone models, to aid in surgery planning can be developed using various publicly available programming environments and languages, for example,, Matlab 7.3 and Matlab Compiler 4.5, C++ or Java. In certain embodiments, the computer software program can have a user interface that includes one or more of the components identified in **FIG. 10****.** Alternatively, one or more off-the-shelf applications can be used to generate the models, such as SolidWorks, Rhinoceros, 3D Slicer or Amira.

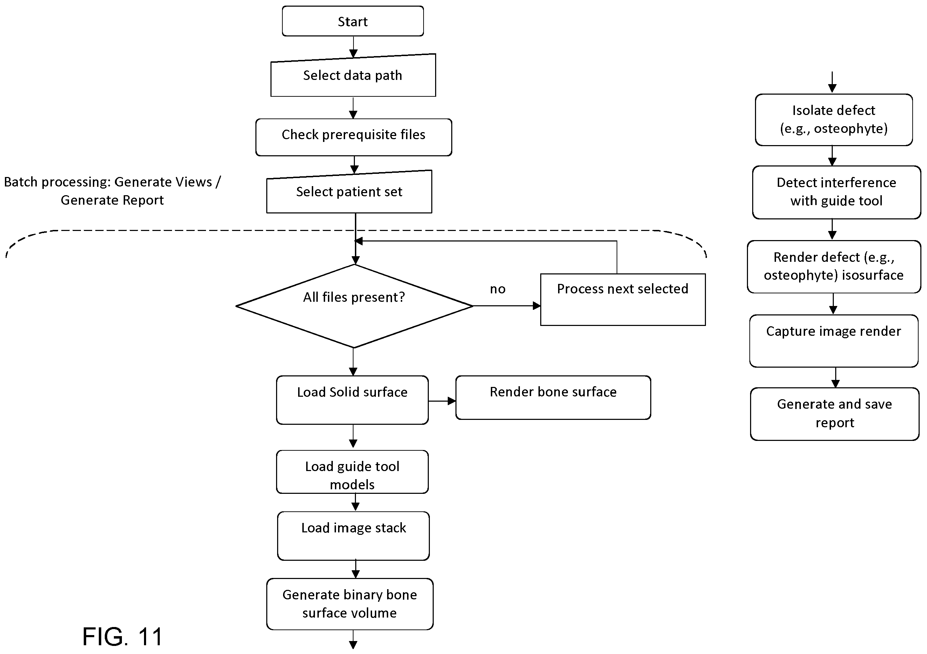

An illustrative flow chart of the high level processes of an exemplary computer software program is shown in **FIG. 11****.** Briefly, a data path associated with one or more patient folders that include data files, for example, patient-specific CT images, solid models, and segmentation images, is selected. The data files associated with the data path can be checked, for example, using file filters, to confirm that all data files are present. For example, in generating models for a knee implant, a data path can confirm the presence of one, several, or all coronal CT data files, sagittal CT data files, a femoral solid model data file, a tibial solid model data file, a femoral guide tool model, a tibial guide tool model, a femoral coronal segmentation model, a femoral sagittal segmentation model, a tibial coronal segmentation model, and a tibial sagittal segmentation model. If the filter check identifies a missing file, the user can be notified. In certain instances, for example, if a tibial or femoral guide tool model file is unavailable, the user may elect to continue the process without certain steps, for example, without guide tool - defect (e.g., osteophyte) interference analysis.

Next, a patient specific *bone-surface model* is obtained and/or rendered. The bone surface model provides basic patient-specific features of the patient's biological structure and serves as a reference for comparison against a model or value that includes the defect(s) of interest. As an illustrative example, previously generated patient-specific files, for example, STL files exported from "SOLID" IGES files in SolidWorks, can be loaded, for example, as triangulation points with sequence indices and normal vectors. The triangles then can be rendered (e.g., using Matlab TRISURF function) to supply or generate the bone-surface model. The bone surface model can include corrections of defects, such as osteophytes removed from the bone. In a similar fashion, one or more guide tool models can be obtained and/or rendered.

Next, a patient specific model or values of the patient's biological feature that include the defect of interest can be obtained and/or rendered. For example, patient-specific defects, such as osteophytes, can be identified from analysis of the patient's segmentation images and corresponding CT scan images. The transformation matrix of scanner coordinate space to image matrix space can be calculated from image slice positions (e.g., the first and last image slice positions). Then, patient-specific segmentation images for the corresponding scan direction can be assessed, along with CT image slices that correspond to the loaded segmentation images. Images can be processed slice by slice and, using selected threshold values (e.g., intensity thresholds, Hounsfield unit thresholds, or neighboring pixel/voxel value thresholds), pixels and/or voxels corresponding to the defects of interest (e.g., osteophytes) can be identified. The identified voxels can provide a *binary bone surface volume* that includes the defects of interest as part of the surface of the patient's biological structure. Various masks can be employed to mask out features that are not of interest, for example, an adjacent biological surface. In some instances, masking can generate apparent unattached portions of an osteophyte defect, for example, when a mask covers a portion of an osteophyte extension.

Next, the defects of interest are isolated by comparing the model that does not include the defects of interest (e.g., *bone-surface model*) with the model or value that does include the defects of interest (e.g., the *binary bone surface volume*)*.* For example, the triangulation points of the bone surface model can be transformed onto an image volume space to obtain a binary representation of the model. This volume binary can be dilated and thinned to obtain a *binary bone model.* The binary bone model then can serve as a mask to the *binary bone surface volume* to identify defect volume separate from the *binary bone surface volume.* For example, for osteophyte detection, the osteophyte volume (e.g., *osteophyte binary volume*), as well as the osteophyte position and attachment surface area, can be distinguished from the patient's biological structure using this comparative analysis. Various thresholds and filters can be applied to remove noise and/or enhance defect detection in this step. For example, structures that have less than a minimum voxel volume (e.g., less than100 voxels) can be removed. Alternatively, or in addition, rules can be added to "reattach" any portion of an osteophyte defect that appears unattached, e.g., due to a masking step.

In an alternative approach, surface data can be used instead of voxel or volume data when comparing the bone surface model with corrected defects and the patient's actual bone surface. The bone surface model, for example, can be loaded as a mesh surface (e.g. in an STL file) or a parametric surface (e.g. in an IGES file) without conversion to volumetric voxel data. The patient's natural bone surface can be derived from the medical image data (e.g. CT data) using, for example, a marching cubes or isosurface algorithm, resulting in a second surface data set. The bone surface model and the natural bone surface can be compared, for example, by calculating intersection between the two surfaces.

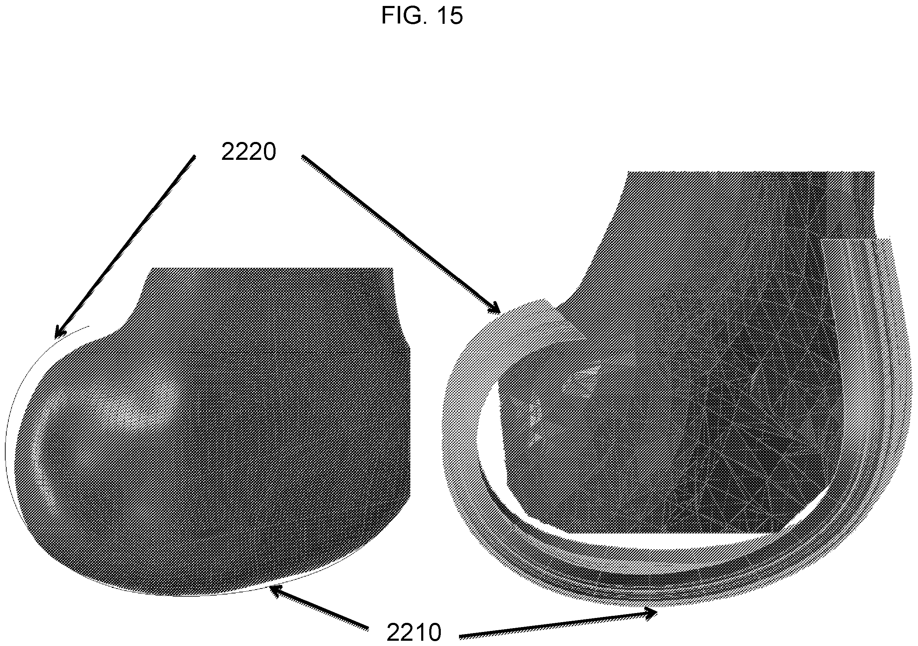

Next, optionally, the models can be used to detect interference between any defect volume and the placement of one or more guide tools and/or implant components. For example, guide tool model triangulation points can be transformed onto an image volume space to obtain a binary representation of the guide tool. The binary structure then can be manipulated (e.g., dilated and eroded using voxel balls having pre-set diameters) to obtain a solid field mask. The solid field mask can be compared against the defect volume, for example, the *osteophyte binary volume,* to identify interfering defect volume, for example, *interfering osteophyte binary volume.* In this way, interfering defect volume and non-interfering defect volume can be determined (e.g., using Matlab ISOSURFACE function), for example, using representative colors or some other distinguishing features in a model. See, e.g., **FIGS. 13** and **15****.** The resulting model image can be rendered on a virtual rendering canvas (e.g., using Matlab GETFRAME function) and saved onto a computer-readable medium.

Finally, optionally, as exemplified by **FIGS. 6 and 7** and by **FIGS. 8 and 9****,** one or more combinations of model features can be combined into one or models or sets of models that convey desired information to the surgeon or clinician. For example, patient-specific bone models can be combined with any number of defects or defect types, any number of resection cuts, any number of guide tools, and/or any number of implant components to convey as much information as desired to the surgeon or clinician. The patient-specific bone model can model any biological structure, for example, any one or more (or portion of) a femoral head and/or an acetabulum; a distal femur, one or both femoral condyle(s), and/or a tibial plateau; a trochlea and/or a patella; a glenoid and/or a humeral head; a talar dome and/or a tibial plafond; a distal humerus, a radial head, and/or an ulna; and a radius and/or a scaphoid. Defects that can be combined with a patient-specific bone model can include, for example, osteophytes, voids, subchondral cysts, articular shape defects (e.g., rounded or flattened articular surfaces or surface portions), varus or valgus deformities, or any other deformities known to those in the art.

The models can include virtual corrections reflecting a surgical plan, such as one or more of removed osteophytes, cut planes, drill holes, realignments of mechanical or anatomical axes. The models can include comparison views demonstrating the anatomical situation before and after applying the planned correction. The individual steps of the surgical plan can also be illustrated in a series of step-by-step images wherein each image shows a different step of the surgical procedure.

The models can be presented to the surgeon as a printed or digital set of images. In another embodiment, the models are transmitted to the surgeon as a digital file, which the surgeon can display on a local computer. The digital file can contain image renderings of the models. Alternatively, the models can be displayed in an animation or video. The models can also be presented as a 3D model that is interactively rendered on the surgeon's computer. The models can, for example, be rotated to be viewed from different angles. Different components of the models, such as bone surfaces, defects, resection cuts, axes, guide tools or implants, can be turned on and off collectively or individually to illustrate or simulate the individual patient's surgical plan. The 3D model can be transmitted to the surgeon in a variety of formats, for example in Adobe 3D PDF or as a SolidWorks eDrawing.

### 4. Parameters as the objective of selection and/or design

In certain embodiments, the assessment process includes selecting and/or designing one or more features and/or feature measurements of an implant component and, optionally, of a corresponding resection cut strategy and/or guide tool that is adapted (e.g., patient-adapted based on one or more of a particular patient's biological features and/or feature measurements) to achieve one or more of the following for the particular patient: (1) correction of a joint deformity; (2) correction of a limb alignment deformity; (3) preservation of bone, cartilage, and/or ligaments at the joint; (4) preservation, restoration, or enhancement of one or more features of the patient's biology, for example, trochlea and trochlear shape; (5) preservation, restoration, or enhancement of joint kinematics, including, for example, ligament function and implant impingement; (6) preservation, restoration, or enhancement of the patient's joint-line location and/or joint gap width; and (7) preservation, restoration, or enhancement of other target features.

Correcting a joint deformity and/or a limb alignment deformity can include, for example, generating a virtual model of the patient's joint, limb, and/or other relevant biological structure(s); virtually correcting the deformity and/or aligning the limb; and selecting and/or designing one or more surgical steps (e.g., one or more resection cuts), one or more guide tools, and/or one or more implant components, to physically perform and/or accommodate the correction.

Preserving bone, cartilage, and/or ligaments can include, for example, identifying diseased tissue from one or more images of the patient's joint, identifying a minimum implant thickness for the patient (based on, for example, femur and/or condyle size and patient weight); virtually assessing combinations of resection cuts and implant component bone cut numbers, angles, and/or orientations; identifying a combination of resection cut and implant component bone cut numbers, angles, and/or orientations that remove the diseased tissue, provide maximum bone preservation and provide at least the minimum implant thickness for the patient; and selecting and/or designing one or more surgical steps (e.g., one or more resection cuts), one or more guide tools, and/or one or more implant components to provide the resection cuts and/or implant component bone cuts that provide removal of the diseased tissue, maximum bone preservation, and at least the minimum implant thickness for the patient.

Preserving or restoring one or more features of a patient's biology can include, for example, selecting and/or designing one or more surgical steps (e.g., one or more resection cuts), one or more guide tools, and/or one or more implant components so that one or more of the patient's postoperative implant features substantially match the patient's preoperative biological features or the patient's healthy biological features (e.g., as identified from a previous image of the patient's joint when it was healthy or from an image the patient's contralateral healthy joint).

Enhancing one or more features of a patient's biology can include, for example, selecting and/or designing one or more surgical steps (e.g., one or more resection cuts), one or more guide tools, and/or one or more implant components so that the postoperative implant includes features that provide a healthy biological surface estimated for the particular patient and/or that provide proper kinematics to the patient's joint.

Preservation or restoration of the patient's joint kinematics can include, for example, selecting and/or designing one or more surgical steps (e.g., one or more resection cuts), one or more guide tools, and/or one or more implant components so that the patient's post-operative joint kinematics substantially match the patient's pre-operative joint kinematics and/or substantially match the patient's healthy joint kinematics (e.g., as identified from previous images of the patient's joint when it was healthy or from an image the patient's contralateral healthy joint).

Enhancing the patient's joint kinematics can include, for example, selecting and/or designing one or more surgical steps (e.g., one or more resection cuts), one or more guide tools, and/or one or more implant components that provide healthy joint kinematics estimated for the particular patient and/or that provide proper joint kinematics to the patient. Optimization of joint kinematics also can include ligament loading or ligament function during motion.

Preservation or restoration of the patient's joint-line location and/or joint gap width can include, for example, selecting and/or designing one or more surgical steps (e.g., one or more resection cuts), one or more guide tools, and/or one or more implant components so that the patient's joint-line and or joint-gap width substantially match the patient's existing joint-line and or joint-gap width or the patient's healthy joint-line and/or joint-gap width (e.g., as identified from previous images of the patient's joint when it was healthy or from an image the patient's contralateral healthy joint).

Enhancing the patient's joint-line location and/or joint gap width can include, for example, selecting and/or designing one or more surgical steps (e.g., one or more resection cuts), one or more guide tools, and/or one or more implant components that provide a healthy joint-line location and/or joint gap width and/or estimated for the particular patient and/or that provide proper kinematics to the patient.

### 5. Multiparametric assessments

Assessment of the above-identified parameters, optionally with one or more additional parameters, can be conducted using various formats. For example, the assessment (e.g., selection and/or design) of one or more parameters can be performed in series, in parallel, or in a combination of the two, optionally with a software-directed computer. For example, one or more implant component features and/or feature measurements, optionally with one or more resection cut features and/or feature measurements and one or more guide tool features and/or feature measurements can be altered and assessed in a series, in parallel, or in a combination format, to assess the fit between selected parameter thresholds and one or more selected features and/or feature measurements. Any one or more of the parameters and features and/or feature measurements can be the first to be selected and/or designed. Alternatively, one or more, or all, of the parameters and/or features can be assessed simultaneously.

The assessment process can be iterative in nature. For example, one or more first parameters can be assessed and related features and/or feature measurements tentatively or conditionally determined. Next, one or more second parameters can be assessed and, optionally, one or more features and/or feature measurements determined. Then, the tentative or conditional features and/or feature measurements for the first assessed parameter(s) optionally can be altered based on the assessment and optional determinations for the second assessed parameters. The assessment process can be fully automated or it can be partially automated allowing for user interaction. User interaction can be particularly useful for quality assurance purposes.

In the assessment, different weighting can be applied to any of the parameters or parameter thresholds, for example, based on the patient's age, the surgeon's preference or the patient's preference. Feedback mechanisms can be used to show the user or the software the effect that certain feature and/or feature measurement changes can have on desired changes to parameters values, e.g., relative to selected parameter thresholds. For example, a feedback mechanism can be used to determine the effect that changes in features intended to maximize bone preservation (e.g., implant component bone cut number, angles and orientation, and related resection cut number, angles, and orientation) have on other parameters such as limb alignment, deformity correction, and/or joint kinematic parameters, for example, relative to selected parameter thresholds. Accordingly, implant component features and/or feature measurements (and, optionally, resection cut and guide tool features and/or feature measurements) can be modeled virtually and modified reiteratively to achieve an optimum solution for a particular patient.

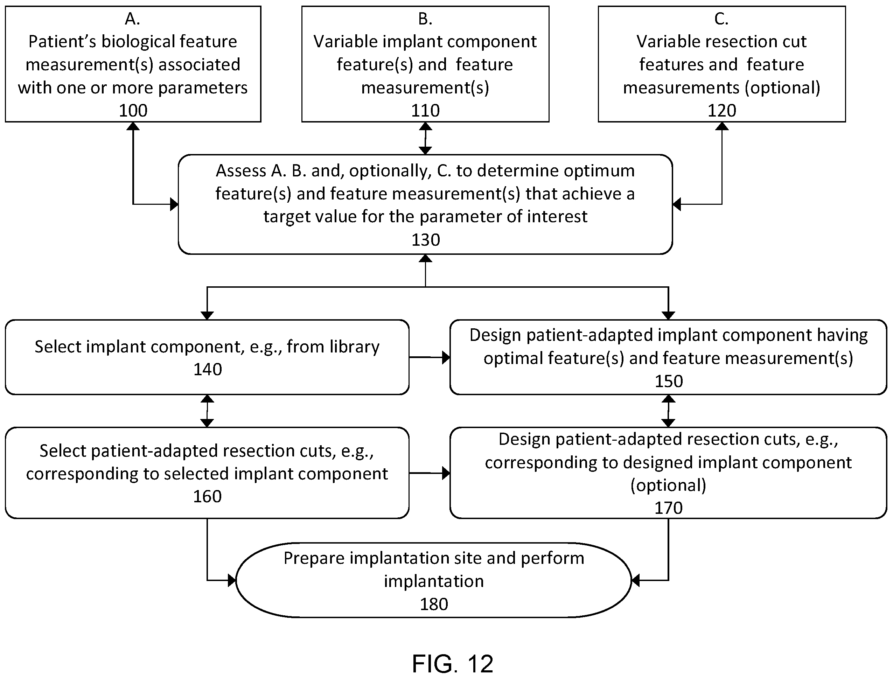

**FIG. 12** is a flow chart illustrating the process of assessing and selecting and/or designing one or more implant component features and/or feature measurements, and, optionally assessing and selecting and/or designing one or more resection cut features and feature measurements, for a patient receiving an implant component. Using the techniques described herein or those suitable and known in the art, one or more of the patient's biological features and/or feature measurements are obtained **100.** In addition, one or more variable implant component features and/or feature measurements are obtained **110.** Optionally, one or more variable resection cut features and/or feature measurements are obtained **120.** Moreover, one or more variable guide tool features and/or feature measurements also can optionally be obtained. Each one of these step can be repeated multiple times, as desired.

The obtained patient's biological features and feature measurements, implant component features and feature measurements, and, optionally, resection cut and/or guide tool features and/or feature measurements then can be assessed to determine the optimum implant component features and/or feature measurements, and optionally, resection cut and/or guide tool features and/or feature measurements, that achieve one or more target or threshold values for parameters of interest **130.** As noted, parameters of interest can include, for example, one or more of (1) joint deformity correction; (2) limb alignment correction; (3) bone, cartilage, and/or ligaments preservation at the joint; (4) preservation, restoration, or enhancement of one or more features of the patient's biology, for example, trochlea and trochlear shape; (5) preservation, restoration, or enhancement of joint kinematics, including, for example, ligament function and implant impingement; (6) preservation, restoration, or enhancement of the patient's joint-line location and/or joint gap width; and (7) preservation, restoration, or enhancement of other target features. This step can be repeated as desired. For example, the assessment step 130 can be reiteratively repeated after obtaining various feature and feature measurement information **100, 110, 120.**

Once the one or more optimum implant component features and/or feature measurements having the optimum features and/or feature measurements can be selected **140,** designed **150,** or selected and further designed **140, 150.** For example, an implant component having some optimum features and/or feature measurements can be designed further using one or more CAD software programs to optimize additional features or feature measurements of the implant component. One or more manufacturing techniques described herein or known in the art can be used in the design step to produce the additional optimized features and/or feature measurements. This process can be repeated as desired.

Optionally, one or more resection cut features and/or feature measurements can be selected **160,** designed **170,** or selected and further designed **160, 170.** For example, a resection cut strategy selected to have some optimum features and/or feature measurements can be designed further using one or more CAD software programs to optimize additional features or measurements of the resection cuts, for example, so that the resected surfaces substantially match optimized bone-facing surfaces of the selected and designed implant component. This process can be repeated as desired.

Moreover, optionally, one or more guide tool features and/or feature measurements can be selected, designed, or selected and further designed. For example, a guide tool having some optimum features and/or feature measurements can be designed further using one or more CAD software programs to optimize additional features or feature measurements of the guide tool. One or more manufacturing techniques described herein or known in the art can be used in the design step to produce the additional, optimized features and/or feature measurements, for example, to facilitate one or more resection cuts that, optionally, substantially match one or more optimized bone-facing surfaces of a selected and designed implant component. This process can be repeated as desired.

As will be appreciated by those of skill in the art, the process of selecting and/or designing an implant component feature and/or feature measurement, resection cut feature and/or feature measurement, and/or guide tool feature and/or feature measurement can be tested against the information obtained regarding the patient's biological features, for example, from one or more MRI or x-ray images from the patient, to ensure that the features and/or feature measurements are optimum with respect to the selected parameter targets or thresholds. Testing can be accomplished by, for example, superimposing the implant image over the image for the patient's joint. Once optimum features and/or feature measurements for the implant component, and optionally for the resection cuts and/or guide tools, have been selected and/or designed, the implant site can he prepared, for example by removing cartilage and/or resecting bone from the joint surface, and the implant component can be implanted into the joint **180.**

The joint implant component bone-facing surface, and optionally the resection cuts and guide tools, can be selected and/or designed to achieve an anatomic or near anatomic fit with the existing surface or with a resected surface of the joint. Moreover, the joint implant component joint-facing surface, and optionally the resection cuts and guide tools, can be selected and/or designed, for example, to replicate the patient's existing joint anatomy, to replicate the patient's healthy joint anatomy, to enhance the patient's joint anatomy, and/or to optimize fit with an opposing implant component. Accordingly, both the existing surface of the joint and the desired resulting surface of the joint can be assessed. This technique can be particularly useful for implants that are not anchored into the bone.

As will be appreciated by those of skill in the art, the physician, or other person practicing the invention, can obtain a measurement of a biological feature (e.g., a target joint) **100** and then directly select **140,** design, **150,** or select and design **140, 150** a joint implant component having desired patient-adapted features and/or feature measurements.

### 5.1 Optimizing features to achieve parametric thresholds

As described herein, certain embodiments can apply modeling, for example, mathematical modeling, to find an optimum solution for selected parameter(s) and/or parameter measurement(s). In particular, a model of a patient's biological feature, for example, a model of patient's joint or limb, can be used to identify, select, and/or design one or more optimum, patient- features and/or feature measurements relative to selected parameters for an implant component and, optionally, for corresponding resection cuts and/or guide tools. A physician or other user can select one or more parameters, parameter thresholds or values, and/or relative weightings for the parameters included in the model. Alternatively or in addition, clinical data, for example obtained from clinical trials, or intraoperative data, can be included in determining optimum features and/or feature measurements for an implant component, resection cut, and/or guide tool.

Certain embodiments described herein include generating and/or using a model, for example, a virtual model, of the patient's joint that includes selected parameters and/or parameter measurements and virtually selecting and/or designing one or more implant components, and optionally resection cuts and/or guide tools to fit the virtual model in accordance with the selected parameters and/or parameter measurements. This approach allows for iterative selection and/or design improvement and can include steps to virtually assess fit relative to the selected parameters and/or parameter measurements, such as (1) correction of a joint deformity; (2) correction of a limb alignment deformity; (3) preservation of bone, cartilage, and/or ligaments at the joint; (4) preservation, restoration, or enhancement of one or more features of the patient's biology, for example, trochlea and trochlear shape; (5) preservation, restoration, or enhancement of joint kinematics, including, for example, ligament function and implant impingement; (6) preservation, restoration, or enhancement of the patient's joint-line location and/or joint gap width; and (7) preservation, restoration, or enhancement of other target features.

One or more parametric thresholds and/or weightings can be applied for the selection and/or designing process. Different parameters can have the same weighting or they can have different weightings. A parameter can include one or more thresholds for selecting one or more implants. The thresholds can include one or more minimum threshold values (e.g., with different weightings), for example, 80%, greater than 80%, 85%, greater than 85%, 90%, greater than 90%, 95%, greater than 95%, 97%, greater than 97%, 98%, greater than 98%, 99%, greater than 99%, 100%, and/or greater than 100% a target value, such as minimum implant coverage of a certain surface on the patient's anatomical structure. Alternatively or in addition, the thresholds can include one or more maximum threshold values (e.g., with different weightings), such as 105%, less than 105%, 103%, less than 103%, 102%, less than 102%, 101 %, less than 101 %, 100%, and/or less than 100% a target value, such as maximum implant coverage of a certain surface on the patient's anatomical structure.

One or more parameter thresholds can be absolute, for example, by selecting and/or designing for only implants that meet the threshold, for example, a threshold for a particular patient of 95% mediolateral femoral condyle coverage all around the condyle(s). An example of a selection and/or design process having multiple absolute thresholds is a process that selects and/or designs femoral implant components that must meet both a minimum threshold for a particular patient of 95% mediolateral femoral condyle coverage in the central weight-bearing region, and a minimum threshold of greater than 80% mediolateral femoral condyle coverage outside the weight-bearing area.

Alternatively or in addition, one or more parameter thresholds can be contingent on one or more other factors. In particular, a selection and/or designing process can successively search a library for implants based on contingent thresholds. For example, femoral implant components meeting a minimum threshold of 99% mediolateral femoral condyle coverage initially can be selected. If no implant meets the threshold, or if some implants meet the threshold but do not meet other parameter thresholds, then a second selection round can include implants meeting a minimum threshold of 98% mediolateral femoral condyle coverage. The process can continue to use additional, contingent thresholds until an implant with the selected parameter thresholds is identified.

Different thresholds can be defined in different anatomic regions and for different parameters. For example, in certain embodiments of a knee implant design, the amount of mediolateral tibial implant component coverage can be set at 90%, while the amount of anteroposterior tibial implant component coverage can be set at 85%. In another illustrative example, the congruency in intercondylar notch shape can be set at 80% required, while the required mediolateral condylar coverage can be set at 95%.

Any of the methods described herein can be performed, at least in part, using a computer-readable medium having instructions stored thereon, which, when executed by one or more processors, causes the one or more processors to perform one or more operations corresponding to one or more steps in the method. Any of the methods can include the steps of receiving input from a device or user and producing an output for a user, for example, a clinician or technician. Executed instructions on the computer readable medium (i.e., a software program) can be used, for example, to receive as input patient-specific information (e.g., images of a patient's biological structure) and provide as output a virtual model of the patient's biological structure. Similarly, executed instructions on a computer-readable medium can be used to receive as input patient-specific information and user-selected and/or weighted parameters and then provide as output to a user values or ranges of values for those parameters. For example, in certain embodiments, patient-specific information can be input into a computer software program for designing one or more resection cuts, guide tools, and/or implant components, and one or more of the following parameters can be optimization in the design process: (1) correction of joint deformity; (2) correction of a limb alignment deformity; (3) preservation of bone, cartilage, and/or ligaments at the joint; (4) preservation, restoration, or enhancement of one or more features of the patient's biology, for example, trochlea and trochlear shape; (5) preservation, restoration, or enhancement of joint kinematics, including, for example, ligament function and implant impingement; (6) preservation, restoration, or enhancement of the patient's joint-line location and/or joint gap width; and (7) preservation, restoration, or enhancement of other target features. As an illustrative example, in certain embodiments, patient-specific information (e.g., radiographic images) can be inputted into the computer program and parameters such as tibial resection cut height (preferably minimize); position of joint-line (preferably preserve for natural kinematics); and thickness of femoral resection cuts (preferably minimize) can be weighted for optimization. As output, one or more resection cuts, guide tools, and/or implant components with the identified parameters optimized can be provided.

Optimization of multiple parameters may result in conflicting constraints; for example, optimizing one parameter may cause an undesired deviation to one or more other parameters. In cases where not all constraints can be achieved at the same time, parameters can be assigned a priority or weight in the software program. The priority or weighting can be automated (e.g., part of the computer program) and/or it can be selected by a user depending on the user's desired design goals, for example, minimization of bone loss, or retention of existing joint-line to preserve kinematics, or combination to accommodate both parameters in overall design.

In any automated process or process step performed by the computer system, constraints pertaining to a specific implant model, to a group of patients or to the individual patient may be taken into account. For example, the maximum implant thickness or allowable positions of implant anchors may depend on the type of implant. The minimum implant thickness can depend on the patient's bone quality.

Any one or more steps of the assessment, selection, and/or design may be partially or fully automated, for example, using a computer-run software program and/or one or more robots. For example, processing of the patient data, the assessment of biological features and/or feature measurements, the assessment of implant component features and/or feature measurements, the optional assessment of resection cut and/or guide tool features and/or feature measurements, the selection and/or design (including the manufacture) of one or more features of a patient-adapted implant component, and/or the implantation procedure(s) may be partially or wholly automated. For example, patient data, with optional user-defined parameters, may be inputted or transferred by a user and/or by electronic transfer into a software-directed computer system that can identify variable implant component features and/ or feature measurements and performs operations to generate one or more virtual models and/or implant design specifications, for example, in accordance with one or more target or threshold parameters. Implant selection and/or design data, with optional user-defined parameters, may be inputted or transferred by a user and/or by electronic transfer into a software-directed computer system that performs a series of operations to transform the data and optional parameters into one or more implant manufacturing specifications. Implant design data or implant manufacturing data, optionally with user-defined parameters, may be inputted or transferred by a user and/or by electronic transfer into a software-directed computer system that directs one or more manufacturing instruments to produce one or more implant components from a starting material, such as a raw material or starting blank material. Implant design data, implant manufacturing data, or implant data, optionally with user-defined parameters, may be inputted or transferred by a user and/or by electronic transfer into a software-directed computer system that performs a series of operations to transform the data and optional parameters into one or more surgical procedure specifications. Implant design data, implant manufacturing data, implant data, or surgical procedure data, optionally with user-defined parameters, may be inputted or transferred by a user and/or by electronic transfer into a software-directed computer system that directs one or more automated surgical instruments, for example, a robot, to perform one or more surgical steps. In certain embodiments, one or more of these actions can be performed as steps in a single process by one or more software-directed computer systems.

In certain embodiments, the final implant component includes one or more bone cuts on its bone-facing surface. The cut planes for these bone cuts can be automatically determined by the computer system, for example using anatomical landmarks. In certain embodiments, a computer program can aid in the determination of bone cuts that are optimized to preserve the maximum amount of bone for each individual patient based on a series of two-dimensional images or a three-dimensional representation of the articular anatomy and geometry and the desired limb alignment and/or desired deformity correction. Optionally, the cut planes can be adjusted by the operator.

The computer system can also construct the implant surfaces. Surfaces may be composed of different elements. In certain embodiments, elements of the surfaces conform to the patient's anatomy. In these situations, the computer system can build a surface using the patient's anatomical model, for example by constructing a surface that is identical with or mostly parallel to the patient's anatomical surface. In certain embodiments, the computer system uses geometric elements such as arcs or planes to construct a surface. Transitions between surfaces can be smoothed using tapers or fillets. Additionally, the computer system may take into account constraints such as minimum or maximum thickness or length or curvature of parts or features of the implant component when constructing the surfaces.

In another embodiment, the computer system can automatically or semiautomatically add other features to the implant design. For example, the computer system can add pegs or anchors or other attachment mechanisms. The system can place the features using anatomical landmarks. Constraints can be used to restrict the placement of the features. Examples of constraints for placement of pegs are the distance between pegs and from the pegs to the edge of the implant, the height of the pegs that results from their position on the implant, and forcing the pegs to be located on the center line. Optionally, the system can allow the user to fine-tune the peg placement, with or without enforcing the constraints.

In another embodiment, the process of selecting an implant component includes selecting one or more component features that optimizes fit with another implant component. In particular, for an implant that includes a first implant component and a second implant component that engage, for example, at a joint interface, selection of the second implant component can include selecting a component having a surface that provides best fit to the engaging surface of the first implant component. For example, for a knee implant that includes a femoral implant component and a tibial implant component, one or both of components can be selected based, at least in part, on the fit of the outer, joint-facing surface with the outer-joint facing surface of the other component. The fit assessment can include, for example, selecting one or both of the medial and lateral tibial grooves on the tibial component and/or one or both of the medial and lateral condyles on the femoral component that substantially negatively-matches the fit or optimizes engagement in one or more dimensions, for example, in the coronal and/or sagittal dimensions. For example, a surface shape of a non-metallic component that best matches the dimensions and shape of an opposing metallic or ceramic or other hard material component. By performing this component matching, component wear can be reduced.

For example, if a metal backed tibial component is used with a polyethylene insert or if an all polyethylene tibial component is used, the polyethylene will typically have one or two curved portions typically designed to mate with the femoral component in a low friction form. This mating can be optimized by selecting a polyethylene insert that is optimized or achieves an optimal fit with regard to one or more of: depth of concavity, width of concavity, length of concavity, radius of concavity, distance between two (medial and lateral concavities).

For example the distance between a medial tibial concavity and a lateral tibial concavity can be selected so that it matches or approximates the distance between a medial and a lateral implant condyle component.

Not only the distance between two concavities, but also the radius can be selected or designed so that it best matches the tangent radius / radii of curvature on the femoral component. A medial and a lateral femoral condyle and opposite tibial component(s) can have a single tangent radius in one or more dimensions, e.g. a coronal plane. They can also have multiple tangent radii of curvature. The tangent radius or radii of curvature on the medial condyle and/or lateral tibial component can be different from that / those on a lateral condyle and/or lateral tibial component.

Similar matching of polyethylene or other plastic shape to opposing metal or ceramic component shape can be performed in the shoulder, e.g. with a glenoid component, or in a hip, e.g. with an acetabular cup, or in an ankle, e.g. with a talar component.

Using patient-specific features and feature measurements, and selected parameters and parameter thresholds, an implant component, resection cut strategy, and/or guide tool can be selected (e.g., from a library) or designed (e.g. virtually designed and manufactured) to have one or more patient-adapted features. Alternatively, the patient-specific features and measurements and the selected parameters and parameter thresholds can be used to select and design an implant component, resection cut strategy, and/or guide tool. For example, an implant component having features that achieve certain parameter thresholds but having other features that do not achieve other parameter thresholds (e.g., a blank feature or a smaller or larger feature can be selected, for example, from a library of implant components. The selected component then can be further designed (e.g. virtually designed and machined) to alter the blank or smaller or larger feature to be achieve the selected parameter, for example, a patient-specific or patient-engineered feature or feature measurement.

### 6. Generating an articular repair system

The articular repair system (e.g., resection cut strategy, guide tools, and implant components) can be formed or selected so that it will achieve a near anatomic fit or match with the surrounding or adjacent cartilage, subchondral bone, menisci and/or other tissue. The shape of the repair system can be based on the analysis of an electronic image (e.g., MRI, CT, digital tomosynthesis, optical coherence tomography or the like). If the articular repair system is intended to replace an area of diseased cartilage or lost cartilage, the near anatomic fit can be achieved using a method that provides a virtual reconstruction of the shape of healthy cartilage in an electronic image.

In one embodiment, a near normal cartilage surface at the position of the cartilage defect can be reconstructed by interpolating the healthy cartilage surface across the cartilage defect or area of diseased cartilage. This can, for example, be achieved by describing the healthy cartilage by means of a parametric surface (e.g. a B-spline surface), for which the control points are placed such that the parametric surface follows the contour of the healthy cartilage and bridges the cartilage defect or area of diseased cartilage. The continuity properties of the parametric surface will provide a smooth integration of the part that bridges the cartilage defect or area of diseased cartilage with the contour of the surrounding healthy cartilage. The part of the parametric surface over the area of the cartilage defect or area of diseased cartilage can be used to determine the shape or part of the shape of the articular repair system to match with the surrounding cartilage.

In another embodiment, a near normal cartilage surface at the position of the cartilage defect or area of diseased cartilage can be reconstructed using morphological image processing. In a first step, the cartilage can be extracted from the electronic image using manual, semi-automated and/or automated segmentation techniques (e.g., manual tracing, region growing, live wire, model-based segmentation), resulting in a binary image. Defects in the cartilage appear as indentations that can be filled with a morphological closing operation performed in 2-D or 3-D with an appropriately selected structuring element. The closing operation is typically defined as a dilation followed by an erosion. A dilation operator sets the current pixel in the output image to 1 if at least one pixel of the structuring element lies inside a region in the source image. An erosion operator sets the current pixel in the output image to 1 if the whole structuring element lies inside a region in the source image. The filling of the cartilage defect or area of diseased cartilage creates a new surface over the area of the cartilage defect or area of diseased cartilage that can be used to determine the shape or part of the shape of the articular repair system to match with the surrounding cartilage or subchondral bone.

As described above, the articular repair system can be formed or selected from a library or database of systems of various sizes, including various medio-lateral (ML) anteroposterior (AP) and supero-inferior(SI) dimensions, curvatures and thicknesses, so that it achieves a near anatomic fit or match with the surrounding or adjacent cartilage, cortical bone, trabecular bone, subchondral bone, as well as cut bone, before or after preparing an implantation site. These systems can be pre-made or made to order for an individual patient. In order to control the fit or match of the articular repair system with the surrounding or adjacent cartilage, cortical bone, trabecular bone, subchondral bone, as well as cut bone before or after preparing an implantation site or menisci and other tissues preoperatively, a software program can be used that projects the articular repair system over the anatomic position where it will be implanted. Suitable software is commercially available and/ or readily modified or designed by a skilled programmer.

In yet another embodiment, the articular surface repair system can be projected over the implantation site prior to, during or after planning or simulating the surgery virtually using one or more 3-D images. The cartilage, cortical bone, trabecular bone, subchondral bone, as well as cut bone, before or after preparing an implantation site and other anatomic structures are extracted from a 3-D electronic image such as an MRI or a CT using manual, semi-automated and/or automated segmentation techniques. In select embodiments, segmentation is not necessary and data are directly displayed using the grayscale image information.

Optionally, a 3-D representation of the cartilage, cortical bone, trabecular bone, subchondral bone, as well as cut bone, before or after preparing an implantation site and other anatomic structures as well as the articular repair system is generated, for example using a polygon or non-uniform rational B-spline (NURBS) surface or other parametric surface representation. For a description of various parametric surface representations see, for example Foley, J. D. et al., Computer Graphics: Principles and Practice in C; Addison-Wesley, 2nd edition, 1995).

The 3D representations of the cartilage, cortical bone, trabecular bone, subchondral bone, as well as cut bone, before or after preparing an implantation site and other anatomic structures and the articular repair system can be merged into a common coordinate system. The articular repair system can then be placed at the desired implantation site. The representations of the cartilage, cortical bone, trabecular bone, subchondral bone, as well as cut bone, before or after preparing an implantation site, menisci and other anatomic structures and the articular repair system are rendered into a 3-D image, for example application programming interfaces (APIs) OpenGL® (standard library of advanced 3-D graphics functions developed by SG), Inc.; available as part of the drivers for PC-based video cards, for example from www.nvidia.com for NVIDIA video cards or www.3dlabs.com for 3Dlabs products, or as part of the system software for Unix workstations) or DirectX® (multimedia API for Microsoft Windows® based PC systems; available from www.microsoft.com). The 3-D image can be rendered showing the cartilage, cortical bone, trabecular bone, subchondral bone, as well as cut bone, before or after preparing an implantation site, menisci or other anatomic objects, and the articular repair system from varying angles, e.g., by rotating or moving them interactively or non-interactively, in real-time or non-real-time.

The software can be designed so that the articular repair system, including surgical tools and instruments with the best fit relative to the cartilage, cortical bone, trabecular bone, subchondral bone, as well as cut bone, before or after preparing an implantation site is automatically selected, for example using some of the techniques described above. Alternatively, the operator can select an articular repair system, including surgical tools and instruments and project it or drag it onto the implantation site using suitable tools and techniques. The operator can move and rotate the articular repair system in three dimensions relative to the implantation site, cut or uncut, and can perform a visual inspection of the fit between the articular repair system and the implantation site, cut or uncut. The visual inspection can be computer assisted. The procedure can be repeated until a satisfactory fit has been achieved. The procedure can be performed manually by the operator; or it can be computer assisted in whole or part. For example, the software can select a first trial implant that the operator can test. The operator can evaluate the fit. The software can be designed and used to highlight areas of poor alignment between the implant and the surrounding cartilage or subchondral bone or menisci or other tissues. Based on this information, the software or the operator can then select another implant and test its alignment. One of skill in the art will readily be able to select, modify and/or create suitable computer programs for the purposes described herein.

In another embodiment, the implantation site can be visualized using one or more cross-sectional 2D images. Typically, a series of 2D cross-sectional images will be used. The 2D images can be generated with imaging tests such as CT, MRI, digital tomosynthesis, ultrasound, or optical coherence tomography using methods and tools known to those of skill in the art. The articular repair system can then be superimposed onto one or more of these 2-D images. The 2-D cross-sectional images can be reconstructed in other planes, e.g., from sagittal to coronal, etc. Isotropic data sets (e.g., data sets where the slice thickness is the same or nearly the same as the in-plane resolution) or near isotropic data sets can also be used. Multiple planes can be displayed simultaneously, for example using a split screen display. The operator can also scroll through the 2D images in any desired orientation in real-time or near-real-time; the operator can rotate the imaged tissue volume while doing this. The articular repair system can be displayed in cross-section utilizing different display planes, e.g., sagittal, coronal or axial, typically matching those of the 2-D images demonstrating the cartilage, cortical bone, trabecular bone, subchondral bone, as well as cut bone, before or after preparing an implantation site, menisci or other tissue. Alternatively or in addition, a three-dimensional display can be used for the articular repair system. The 2D electronic image and the 2D or 3-D representation of the articular repair system can be merged into a common coordinate system. The articular repair system can then be placed at the desired implantation site. The series of 2D cross-sections of the anatomic structures, the implantation site and the articular repair system can be displayed interactively (e.g., the operator can scroll through a series of slices) or noninteractively (e.g., as an animation that moves through the series of slices), in real-time or non-real-time.

In another embodiment, the fit between the implant and the implantation site is evaluated. The implant can be available in a range of different dimensions, sizes, shapes and thicknesses. Different dimensions, sizes, shapes and thicknesses can be available for a medial condyle, a lateral condyle, a trochlea, a medial tibia, a lateral tibia, the entire tibia, a medial patella, a lateral patella an entire patella, a medial trochlea, a central trochlea, a lateral trochlea, a portion of a femoral head, an entire femoral head, a portion of an acetabulum, an entire acetabulum, a portion of a glenoid, an entire glenoid, a portion of a humeral head, an entire humeral head, a portion of an ankle joint an entire ankle joint, a portion or an entire elbow, wrist, hand, finger, spine, facet joint.

In certain embodiment, a combination of parameters can be selected. For example, one or more of an M-L measurement, an A-P measurement, and an S-I measurement o a patient's joint can be obtained from the subject preoperatively, for example, from one or more images of the subject's joint. Then, based on the one or measurements, an implant or implant component for the subject's joint can be designed or selected preoperatively.

### 6.1 Designing and/or selecting a femoral implant component

A traditional femoral implant component used in a primary total knee arthroplasty ("TKA") includes: an outer, joint-facing surface having a standard topography; an inner, bone-facing surface having five standard bone cuts; and a standard implant thickness between the joint-facing surface and the bone-facing surface. **FIG. 13** shows a coronal view of a patient's femoral bone **800** and (in dashed lines) the five standard resection cuts used to remove portions of the subject's distal femur in order to fit the traditional femoral implant component. As shown by the dashed lines in the figure, a traditional resection performed in a TKA includes a horizontal resection cut **810,** an anterior resection cut **820,** a posterior resection cut **830,** an anterior chamfer resection cut **840,** and a posterior chamfer resection cut **850.** The anterior and posterior resection cuts **820, 830** typically are placed in a substantially coronal plane. With a traditional implant, the five standard resection cuts are performed so that the patient's distal femur approximately negatively-matches the standard five bone cuts on the inner, bone-facing surface of the traditional femoral implant component. In other words, the patient's bone is resected to fit the shape of the traditional femoral implant component.

In various embodiments described herein, one or more features of an implant component and/or implant procedure are designed and/or selected so that the implant component fits the patient. For example, in certain embodiments, one or more of an implant component and/or implant procedure are designed and/or selected preoperatively, based on patient-specific data, to substantially match (e.g., substantially negatively-match and/or substantially positively-match) one or more of the patient's biological structures.

The one or more patient-adapted (i.e., patient-specific or patient-engineered) curvatures and standard curvatures on the joint-facing surface of a femoral implant component can be combined in a condyle to take on any overall shape. **FIGS. 14A** through **14F** illustrate exemplary curvatures **880** for one or more condylar coronal or sagittal curvatures. An implant component condyle can include a surface curvature corresponding to a section of any one or more geometric shapes, such as a circle, a parabola, a hyperbola, an ellipsoid, and any other geometric shape. The curvature also can include, in part, a straight line portion, as illustrated by the curvature **880** in **FIG. 14F****.** Different portions of a condyle, such as the anterior portion, the distal portion, the posterior portion, the load-bearing portion, and/or the non-load-bearing portion, each can include a different curvature than one or more other portions. For example, in certain embodiments the load bearing-portion of the medial condyle can include a different curvature than the non-load-bearing portions of the same condyle and optionally, than any or all sections of the lateral condyle. Similarly, in certain embodiments the load bearing-portion of the lateral condyle can include a different curvature than the non-load-bearing portions of the same condyle and optionally, than any or all sections of the medial condyle.

The curvature **880** in **FIG. 14A** corresponds to a section of a circle, and, accordingly, can be defined by a single radius of curvature across its entire curvature. Two exemplary tangent radii of curvature are shown as dotted lines in **FIGS. 14A****.** However, the curvatures **880** in **FIGS. 14B** through **14F** each correspond to a section of a non-circular geometric shape and therefore each include tangent radii of curvature having different lengths across their curvatures. Moreover, the straight line portion of the curvature 880 illustrated in **FIG. 14F** includes no tangent radius of curvature. Accordingly, as exemplified by the circular curvature shown in **FIG. 14A****,** a curvature or a portion of a curvature of an implant component, for example, a curvature or a portion of a curvature on the joint-facing surface of a femoral implant component, such as condylar coronal or sagittal curvature or portions of a curvature, can include a single tangent radius of curvature. Alternatively or in addition, as exemplified by the non-circular curvatures shown in **FIGS. 14B** through **14F****,** a curvature or a portion of a curvature of an implant component, for example, a curvature or a portion of a curvature on the joint-facing surface of a femoral implant component, such as condylar coronal or sagittal curvature, can include multiple tangent radii of curvature and, optionally, no tangent radii of curvature (e.g., for a straight line portion of a curvature).

Moreover, in certain embodiments, a curvature or a portion of a curvature of an implant component, for example, a curvature or a portion of a curvature on the joint-facing surface of a femoral implant component, such as condylar coronal or sagittal curvature, can include a combination of patient-specific tangent radii of curvature, patient-engineered tangent radii of curvature, and/or standard tangent radii of curvature. In other words, a curvature can include a portion that is patient-specific, a portion that is patient-engineered, and/or a portion that is standard. For example, **FIG. 15** illustrates a design for a femoral implant component having a J-curve that is partially patient-specific and partially patient-engineered. Specifically, as shown in the figure, the J-curve is designed to be patient-specific except for a distal portion and a posterior portion of the curvature. In the distal portion **2210,** the J-curve is smoothed relative to the patient's corresponding J-curve. In the posterior portion **2220,** the J-curve is tapered out 1-2 mm relative to the patient's corresponding J-curve, in order to match the height of the patient's remaining posterior cartilage.

### 6.2 Tibial implant component

In various embodiments described herein, one or more features of a tibial implant component are designed and/or selected, optionally in conjunction with an implant procedure, so that the tibial implant component fits the patient. For example, in certain embodiments, one or more features of a tibial implant component and/or implant procedure are designed and/or selected, based on patient-specific data, so that the tibial implant component substantially matches (e.g., substantially negatively-matches and/or substantially positively-matches) one or more of the patient's biological structures. Alternatively or in addition, one or more features of a tibial implant component and/or implant procedure can be preoperatively engineered based on patient-specific data to provide to the patient an optimized fit with respect to one or more parameters, for example, one or more of the parameters described above. For example, in certain embodiments, an engineered bone preserving tibial implant component can be designed and/or selected based on one or more of the patient's joint dimensions as seen, for example, on a series of two-dimensional images or a three-dimensional representation generated, for example, from a CT scan or MRI scan. Alternatively or in addition, an engineered tibial implant component can be designed and/or selected, at least in part, to provide to the patient an optimized fit with respect to the engaging, joint-facing surface of a corresponding femoral implant component.

Certain embodiments include a tibial implant component having one or more patient-adapted (e.g., patient-specific or patient-engineered) features and, optionally, one or more standard features. Optionally, the one or more patient-adapted features can be designed and/or selected to fit the patient's resected tibial surface. For example, depending on the patient's anatomy and desired postoperative geometry or alignment, a patient's lateral and/or medial tibial plateaus may be resected independently and/or at different depths, for example, so that the resected surface of the lateral plateau is higher (e.g., 1 mm, greater than 1 mm, 2 mm, and/or greater than 2 mm higher) or lower (e.g., 1 mm, greater than 1 mm, 2 mm, and/or greater than 2 mm lower) than the resected surface of the medial tibial plateau.

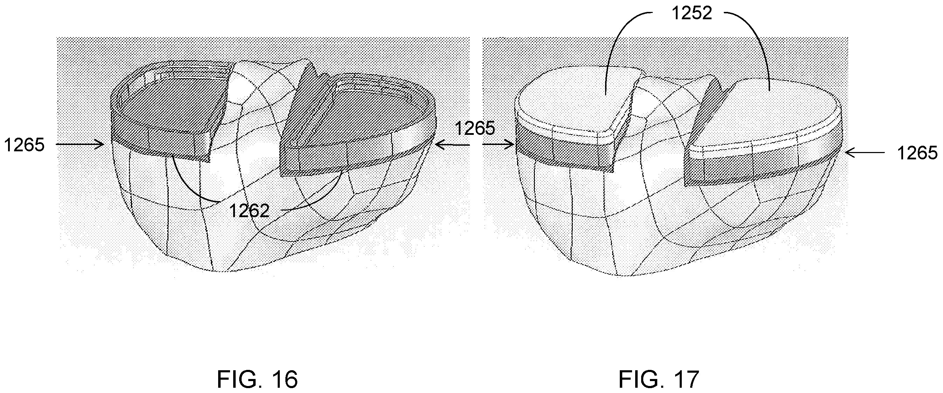

Accordingly, in certain embodiments, tibial implant components can be independently designed and/or selected for each of the lateral and/or medial tibial plateaus. For example, the perimeter of a lateral tibial implant component and the perimeter of a medial tibial implant component can be independently designed and/or selected to substantially match the perimeter of the resection surfaces for each of the lateral and medial tibial plateaus. **FIG. 16** and **FIG. 17** show exemplary unicompartmental medial and lateral tibial implant components without **(****FIG. 16****)** and with **(****FIG. 17****)** a polyethylene layer or insert. As shown, the lateral tibial implant component and the medial tibial implant component have different perimeters shapes, each of which substantially matches the perimeter of the corresponding resection surface **1265.** In addition, the polyethylene layers or inserts **1252** for the lateral tibial implant component and the medial tibial implant component have perimeter shapes that correspond to the respective implant component perimeter shapes. In certain embodiments, one or both of the implant components can be made entirely of a plastic or polyethylene (rather than having a having a polyethylene layer or insert) and each entire implant component can include a perimeter shape that substantially matches the perimeter of the corresponding resection surface.

Moreover, the height of a lateral tibial implant component and the height of a medial tibial implant component can be independently designed and/or selected to maintain or alter the relative heights generated by different resection surfaces for each of the lateral and medial tibial plateaus. For example, the lateral tibial implant component can be thicker (e.g., 1 mm, greater than 1 mm, 2 mm, and/or greater than 2 mm thicker) or thinner (e.g., 1 mm, greater than 1 mm, 2 mm, and/or greater than 2 mm thinner) than the medial tibial implant component to maintain or alter, as desired, the relative height of the joint-facing surface of each of the lateral and medial tibial implant components. As shown in **FIG. 16** and **FIG. 17****,** the relative heights of the lateral and medial resection surfaces **1262** is maintained using lateral and medial implant components (and lateral and medial polyethylene layers or inserts) that have the same thickness. Alternatively, the lateral implant component (and/or the lateral polyethylene layer or insert) can have a different thickness than the medial implant component (and/or the medial polyethylene layer or insert). For embodiments having one or both of the lateral and medial implant components made entirely of a plastic or polyethylene (rather than having a having a polyethylene layer or insert) the thickness of one implant component can be different from the thickness of the other implant component.

Different medial and lateral tibial cut heights also can be applied with a one piece implant component, e.g., a monolithically formed, tibial implant component. In this case, the tibial implant component can have an angled surface or a step connecting the medial and the lateral component. Tibial components also can include the same medial and lateral cut height.

In another embodiment, a mathematical function can be applied to derive a medial implant slope and/or a lateral implant slope, or both (wherein both can be the same). In certain embodiments, the mathematical function can include a measurement derived from one or more of the patient's joint dimensions as seen, for example, on a series of two-dimensional images or a three-dimensional representation generated, for example, from a CT scan or MRI scan. For example, the mathematical function can include a ratio between a geometric measurement of the patient's femur and the patient's tibial slope. Alternatively or in addition, the mathematical function can be or include the patient's tibial slope divided by a fixed value. In certain embodiments, the mathematical function can include a measurement derived from a corresponding implant component for the patient, for example, a femoral implant component, which itself can include patient-specific, patient-engineered, and/or standard features. Many different possibilities to derive the patient's slope using mathematical functions can be applied by someone skilled in the art.

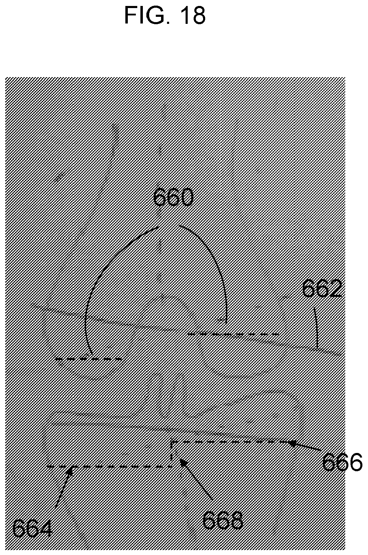

In certain embodiments, the medial and lateral tibial plateau can be resected at the same angle. For example, a single resected cut or the same multiple resected cuts can be used across both plateaus. In other embodiments, the medial and lateral tibial plateau can be resected at different angles. Multiple resection cuts can be used when the medial and lateral tibial plateaus are resected at different angles. Optionally, the medial and the lateral tibia also can be resected at a different distance relative to the tibial plateau. In this setting, the two horizontal plane tibial cuts medially and laterally can have different slopes and/or can be accompanied by one or two vertical or oblique resection cuts, typically placed medial to the tibial plateau components. **FIG. 18** shows several exemplary tibial resection cuts, which can be used in any combination for the medial and lateral plateaus. **FIG. 18** depicts designed and/or selected resection cuts can include a femoral non-coplanar resection cut **660,** a femoral angled cut **662,** a tibial non-coplanar resection cut **664,** a tibial angled cut **666.** Non-coplanar resection cuts also can be angled and can include a step-cut **668** to connect the non-coplanar resection heights.

The medial tibial implant component plateau can have a flat, convex, concave, or dished surface and/or it can have a thickness different than the lateral tibial implant component plateau. The lateral tibial implant component plateau can have a flat, convex, concave, or dished surface and/or it can have a thickness different than the medial tibial implant component plateau. The different thickness can be achieved using a different material thickness, for example, metal thickness or polyethylene or insert thickness on either side. In certain embodiments, the lateral and medial surfaces are selected and/or designed to closely resemble the patient's anatomy prior to developing the arthritic state.

The height of the medial and/or lateral tibial implant component plateau, e.g., metal only, ceramic only, metal backed with polyethylene or other insert, with single or dual inserts and single or dual tray configurations can be determined based on the patient's tibial shape, for example using an imaging test.

Alternatively, the height of the medial and/or lateral tibial component plateau, e.g. metal only, ceramic only, metal backed with polyethylene or other insert, with single or dual inserts and single or dual tray configurations can be determined based on the patient's femoral shape. For example, if the patient's lateral condyle has a smaller radius than the medial condyle and/or is located more superior than the medial condyle with regard to its bearing surface, the height of the tibial component plateau can be adapted and/or selected to ensure an optimal articulation with the femoral bearing surface. In this example, the height of the lateral tibial component plateau can be adapted and/or selected so that it is higher than the height of the medial tibial component plateau.

Alternatively, the height of the medial and/or lateral tibial component plateau, e.g. metal only, ceramic only, metal backed with polyethylene or other insert, with single or dual inserts and single or dual tray configurations can be determined based on the shape of a corresponding implant component, for example, based on the shape of certain features of the patient's femoral implant component. For example, if the femoral implant component includes a lateral condyle having a smaller radius than the medial condyle and/or is located more superior than the medial condyle with regard to its bearing surface, the height of the tibial implant component plateaus can be adapted and/or selected to ensure an optimal articulation with the bearing surface(s) of the femoral implant component. In this example, the height of the lateral tibial implant component plateau can be adapted and/or selected to be higher than the height of the medial tibial implant component plateau.

The perimeter of the tibial component, metal backed, optionally poly inserts, or all plastic or other material, can be matched to or derived from the patient's tibial shape, and can be optimized for different cut heights and/or tibial slopes. In a preferred embodiment, the shape is matched to the cortical bone of the cut surface.

In certain embodiments, a patient-specific A-P slope is used if the patient's anatomic slope is between 0 and 7 degrees and a slope of 7 degrees is used if the patient's anatomic slope is anything over 7 degrees. Someone skilled in the art will recognize other methods for determining the tibial slope and for adapting it during implant and jig design to achieve a desired implant slope.

A different tibial slope can be applied on the medial and the lateral side. A fixed slope can be applied on one side, while the slope on the other side can be adapted based on the patient's anatomy. For example, a medial slope can be fixed at 5 degrees, while a lateral slope matches that of the patient's tibia. In this setting, two unicondylar tibial inserts or trays can be used. Alternatively, a single tibial component, optionally with metal backing, can be used wherein said component does not have a flat, bone-facing surface, but includes, for example, an oblique portion to connect the medial to the lateral side substantially negatively match resected lateral and medial tibial surfaces as shown, for example, in **FIG. 18****.**

The matched shape on the metal backing can serve the purpose of maintaining a minimum polyethylene thickness. It can, however, also include design features to provide a locking mechanism between the polyethylene or other insert and the metal backing. Such locking features can include ridges, edges, or an interference fit. In the case of an interference fit, the polyethylene can have slightly larger dimensions at the undersurface convexity than the matching concavity on the metal tray. This can be stabilized against rails or dove tail locking mechanisms in the center or the sides of the metal backing. Other design options are possible. For example, the polyethylene extension can have a saucer shape that can snap into a matching recess on the metal backing. In addition, any corresponding pieces of the component, such as a metal tray, also can include a matching groove to engage the curved surface of the plastic material.

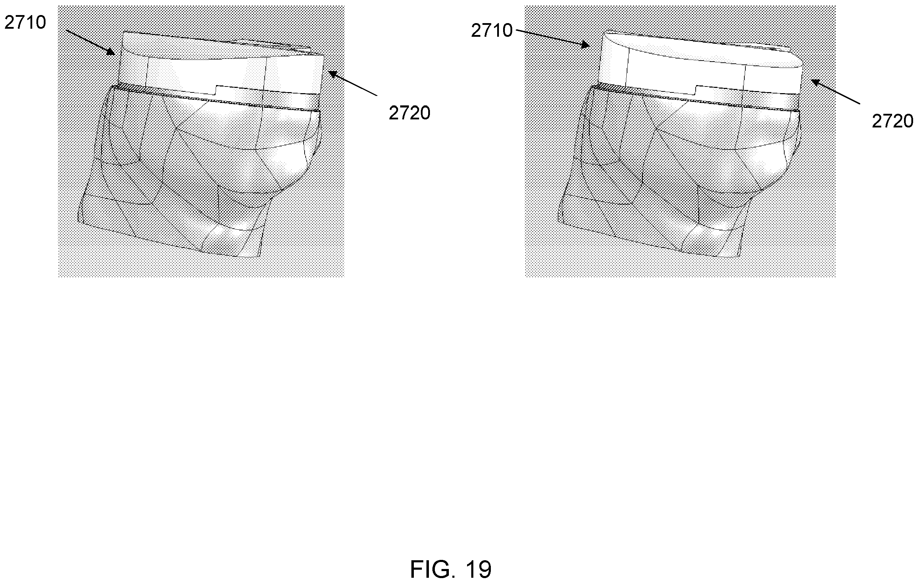

In certain embodiments, the sagittal curvature of the femoral component can be designed to be tilted, as suggested by **FIG. 19****.** The corresponding curvature of the tibial surface can be tilted by that same slope, which can allow for thicker material on the corresponding tibial implant, for example, thicker poly at the anterior **2710** or posterior **2720** aspect of the tibial implant. The femoral component J-curve, and optionally the corresponding curvature for the tibial component, can be tilted by the same slope in both the medial and lateral condyles, just in the medial condyle or just in the lateral condyle or both independently or coupled. In certain embodiments, some additional material can be removed or the material thickness can be adapted from the posterior aspect of the femoral and/or tibial curvatures to allow for rotation.

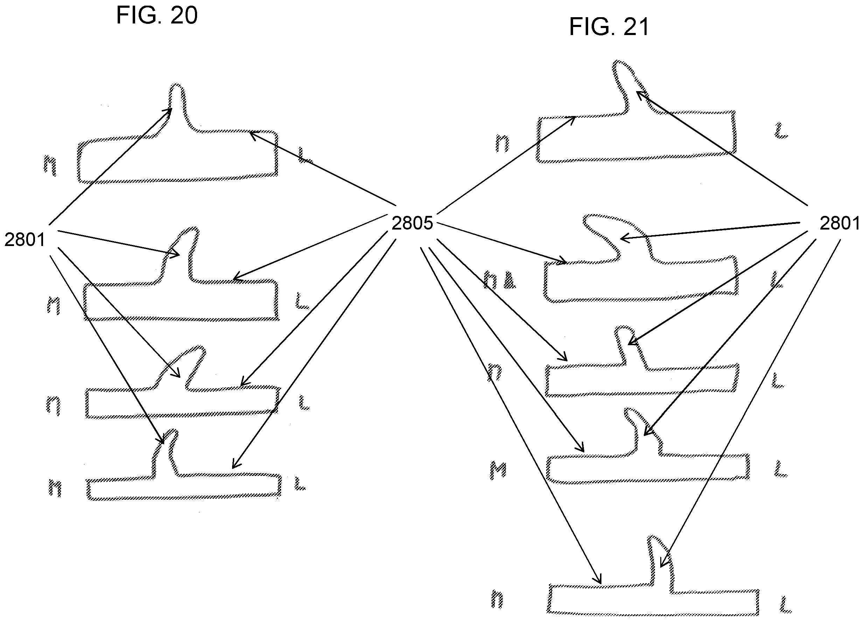

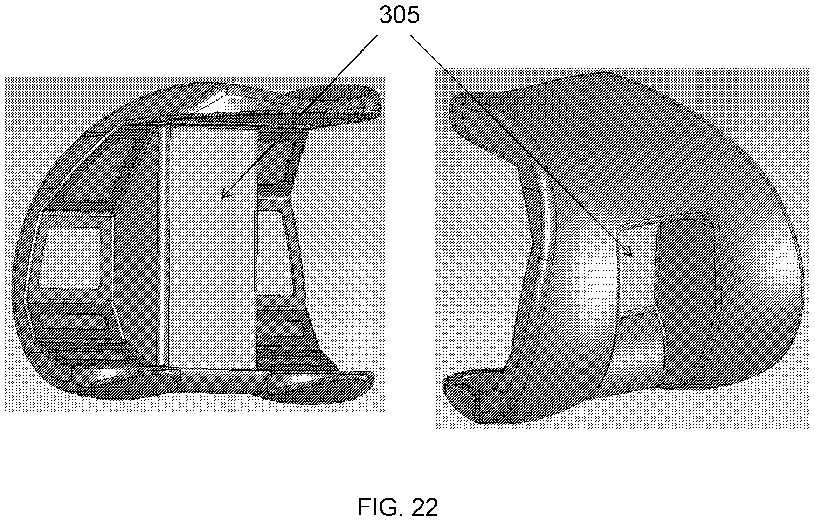

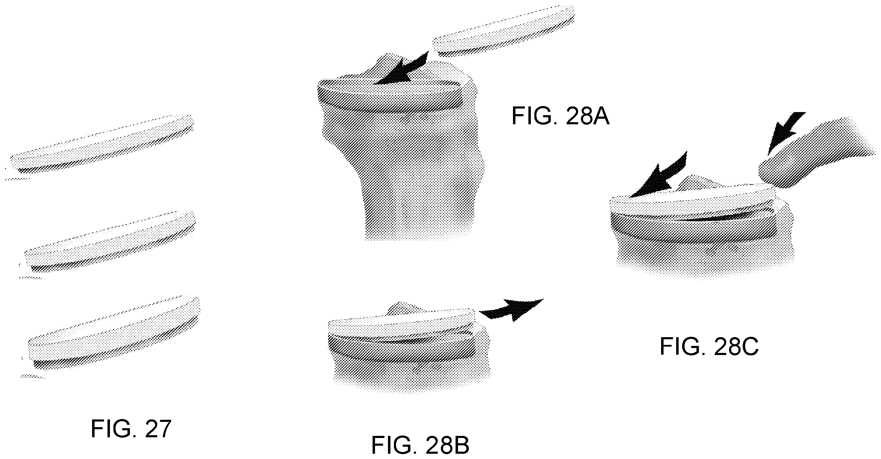

In addition to the implant component features described above, certain embodiments can include features and designs for cruciate substitution. These features and designs can include, for example, a post or projection that projects from the bone-facing surface of the tibial implant component to engage on the corresponding an intercondylar housing, receptacle, or bars on the corresponding femoral implant component. **FIGS. 28** and **29** depict exemplary cross-sections of tibial implant components having a post **2801** projecting from the bone-facing surface **2805** of the implant component. **FIG. 22** depicts various features of the intercondylar housing on a cruciate-retaining femoral implant component. The post or projection **2801** on the tibial implant component shown in **FIG. 20** and **FIG. 21** can be used in conjunction with an intercondylar housing **305,** receptacle, and/or bars on a femoral implant component, shown in **FIG. 22****,** as a substitute for a patient's posterior cruciate ligament ("PCL"), which may be sacrificed during the implant procedure. Specifically, the post or projection on the tibial component engages the intercondylar housing, receptacle or bars on the femoral implant component to stabilize the joint during flexion, particular during deep flexion.

In certain embodiments, the tibial implant component can be designed and manufactured to include the post or projection as a permanently integrated feature of the implant component. However, in certain embodiments, the post or projection can be modular. For example, the post or projection can be designed and/or manufactured separate from the tibial implant component and optionally joined with the component, either prior to (e.g., preoperatively) or during the implant procedure. For example, a modular post or projection and a tibial implant component can be mated using an integrating mechanism such as respective male and female screw threads, other male-type and female-type locking mechanisms, or other mechanism capable of integrating the post or projection into or onto the tibial implant component and providing stability to the post or projection during normal wear. A modular post or projection can be joined to a tibial implant component at the option of the surgeon or practitioner, for example, by removing a plug or other device that covers the integrating mechanism and attaching the modular post or projection at the uncovered integrating mechanism.

The post or projection can include features that are patient-adapted (e.g., patient-specific or patient-engineered). In certain embodiments, the post or projection includes one or more features that are designed and/or selected preoperatively, based on patient-specific data including imaging data, to substantially match one or more of the patient's biological features. For example, the length, width, height, and/or curvature of one or more portions of the post or projection can be designed and/or selected to be patient-specific. Alternatively or in addition, one or more features of the post or projection can be engineered based on patient-specific data to provide to the patient an optimized fit. For example, the length, width, height, and/or curvature of one or more portions of the post or projection can be designed and/or selected to be patient-engineered. One or more thicknesses of the housing, receptacle, or bar can be matched to patient specific measurements. One or more dimensions of the post or projection can be adapted based on one or more implant dimensions (e.g., one or more dimensions of the housing, receptacle or bar on the corresponding femoral implant component), which can be patient-specific, patient-engineered or standard. One or more dimensions of the post or projection can be adapted based on one or more of patient weight, height, sex, and body mass index. In addition, one or more features of the post or projection can be standard.

Different dimensions of the post or projection can be shaped, adapted, or selected based on different patient dimensions and implant dimensions. Examples of different technical implementations are provided in **Table 3.** These examples are in no way meant to be limiting. Someone skilled in the art will recognize other means of shaping, adapting or selecting a tibial implant post or projection based on the patient's geometry including imaging data.

**Table 3: Examples of different technical implementations of a cruciate-sacrificing tibial implant component**

| **Post or projection feature** | **Corresponding patient anatomy, e.g., derived from imaging studies or intraoperative measurements** |
|---|---|
| **Mediolateral width** | Maximum mediolateral width of patient intercondylar notch or fraction thereof |
| **Mediolateral width** | Average mediolateral width of intercondylar notch |
| **Mediolateral width** | Median mediolateral width of intercondylar notch |
| **Mediolateral width** | Mediolateral width of intercondylar notch in select regions, e.g. most inferior zone, most posterior zone, superior one third zone, mid zone, etc. |
| **Superoinferior height** | Maximum superoinferior height of patient intercondylar notch or fraction thereof |
| **Superoinferior height** | Average superoinferior height of intercondylar notch |
| **Superoinferior height** | Median superoinferior height of intercondylar notch |
| **Superoinferior height** | Superoinferior height of intercondylar notch in select regions, e.g. most medial zone, most lateral zone, central zone, etc. |
| **Anteroposterior length** | Maximum anteroposterior length of patient intercondylar notch or fraction thereof |
| **Anteroposterior length** | Average anteroposterior length of intercondylar notch |
| **Anteroposterior length** | Median anteroposterior length of intercondylar notch |
| **Anteroposterior length** | Anteroposterior length of intercondylar notch in select regions, e.g. most anterior zone, most posterior zone, central zone, anterior one third zone, posterior one third zone etc. |

The height or M-L width or A-P length of the intercondylar notch can not only influence the length but also the position or orientation of a post or projection from the tibial implant component.

The dimensions of the post or projection can be shaped, adapted, or selected not only based on different patient dimensions and implant dimensions, but also based on the intended implantation technique, for example, the intended tibial component slope or rotation and/or the intended femoral component flexion or rotation. For example, at least one of an anteroposterior length or superoinferior height can be adjusted if a tibial implant is intended to be implanted at a 7 degrees slope as compared to a 0 degrees slope, reflecting the relative change in patient or trochlear or intercondylar notch or femoral geometry when the tibial component is implanted. Moreover, at least one of an anteroposterior length or superoinferior height can be adjusted if the femoral implant is intended to be implanted in flexion, for example, in 7 degrees flexion as compared to 0 degrees flexion. The corresponding change in post or projection dimension can be designed or selected to reflect the relative change in patient or trochlear or intercondylar notch or femoral geometry when the femoral component is implanted in flexion.

In another example, the mediolateral width can be adjusted if one or both of the tibial and/or femoral implant components are intended to be implanted in internal or external rotation, reflecting, for example, an effective elongation of the intercondylar dimensions when a rotated implantation approach is chosen. Features of the post or projection can be oblique or curved to match corresponding features of the femoral component housing, receptacle or bar. For example, the superior portion of the post projection can be curved, reflecting a curvature in the roof of the femoral component housing, receptacle, or bar, which itself may reflect a curvature of the intercondylar roof. In another example, a side of a post or projection may be oblique to reflect an obliquity of a side wall of the housing or receptacle of the femoral component, which itself may reflect an obliquity of one or more condylar walls. Accordingly, an obliquity or curvature of a post or projection can be adapted based on at least one of a patient dimension or a femoral implant dimension. Alternatively, the post or projection of the tibial implant component can be designed and/or selected based on generic or patient-derived or patient-desired or implant-desired kinematics in one, two, three or more dimensions. Then, the corresponding surface(s) of the femoral implant housing or receptacle can be designed and/or selected to mate with the tibial post or projection, e.g., in the ML plane.

The tibial post or projection can be straight. Alternatively, the tibial post or projection can have a curvature or obliquity in one, two or three dimensions, which can optionally be, at least in part, reflected in the internal shape of the box. One or more tibial projection or post dimensions can be matched to, designed to, adapted to, or selected based on one or more patient dimensions or measurements. Any combination of planar and curved surfaces is possible.

In certain embodiments, the position and/or dimensions of the tibial implant component post or projection can be adapted based on patient-specific dimensions. For example, the post or projection can be matched with the position of the posterior cruciate ligament or the PCL insertion. It can be placed at a predefined distance from anterior or posterior cruciate ligament or ligament insertion, from the medial or lateral tibial spines or other bony or cartilaginous landmarks or sites. By matching the position of the post with the patient's anatomy, it is possible to achieve a better functional result, better replicating the patient's original anatomy.

### 7. Optimizing Soft-Tissue Tension, Ligament Tension, Balancing, Flexion and Extension Gap

The surgeon can, optionally, make adjustments of implant position and/or orientation such as rotation, bone cuts, cut height and selected component thickness, insert thickness or selected component shape or insert shape. In this manner, an optimal compromise can be found, for example, between biomechanical alignment and joint laxity or biomechanical alignment and joint function, e.g., in a knee joint flexion gap and extension gap. Thus, multiple approaches exist for optimizing soft-tissue tension, ligament tension, ligament balance, and/or flexion and extension gap. These include, for example, one or more of the exemplary options described in **Table 4.**

**Table 4: Exemplary approach options for optimizing soft-tissue tension, ligament tension, ligament balance, and/or flexion and extension gap**

| **Option #** | **Description of Exemplary Option** |
|---|---|
| **1** | Position of one or more femoral bone cuts |
| **2** | Orientation of one or more femoral bone cuts |
| **3** | Location of femoral component |
| **4** | Orientation of femoral component, including rotational alignment in axial, sagittal and coronal direction |
| **5** | Position of one or more tibial bone cuts |
| **6** | Orientation of one or more tibial bone cuts including sagittal slope, mediolateral orientation |
| **7** | Location of tibial component |
| **8** | Orientation of tibial component, including rotational alignment in axial, sagittal and coronal direction |
| **9** | Tibial component height |
| **10** | Medial tibial insert or component or composite height |
| **11** | Lateral tibial insert or component or composite height |
| **12** | Tibial component profile, e.g., convexity, concavity, trough, tangent radii of curvature |
| **13** | Medial tibial insert or component or composite profile, e.g. convexity, concavity, trough, tangent radii of curvature |
| **14** | Lateral tibial insert or component or composite profile, e.g. convexity, concavity, trough, tangent radii of curvature |
| **15** | Select soft-tissue releases, e.g. partial or full releases of retinacula and/or ligaments, "pie-crusting" etc. |

Any one option described in **Table 4** can be optimized alone or in combination with one or more other options identified in the table and/or known in the art for achieving different flexion and extension, abduction, or adduction, internal and external positions and different kinematic requirements.

In one embodiment, the surgeon can initially optimize the femoral and tibial resections. Optimization can be performed by measuring soft-tissue tension or ligament tension or balance for different flexion and extension angles or other joint positions before any bone has been resected, once a first bone resection on a first articular surface has been made and after a second bone resection on a first or second articular surface has been made, such as a femur and a tibia, humerus and a glenoid, femur and an acetabulum.

The position and orientation between a first implant component and a second, opposing implant component or a first articular surface and a trial implant or a first trial implant and a second trial implant or an alignment guide and an instrument guide and any combinations thereof can be optimized with the use of, for example, interposed spacers, wedges, screws and other mechanical or electrical methods known in the art. A surgeon may desire to influence joint laxity as well as joint alignment. This can be optimized for different flexion and extension, abduction, or adduction, internal and external rotation angles. For this purpose, spacers can be introduced at or between one or more steps in the implant procedure. One or more of the spacers can be attached or in contact with one or more instruments, trials or, optionally, patient-specific molds. The surgeon can intraoperatively evaluate the laxity or tightness of a joint using spacers with different thicknesses or one or more spacers with the same thickness. For example, spacers can be applied in a knee joint in the presence of one or more trials or instruments or patient-specific molds and the flexion gap can be evaluated with the knee joint in flexion. The knee joint can then be extended and the extension gap can be evaluated. Ultimately, the surgeon selects for a given joint an optimal combination of spacers and trial or instrument or patient specific mold. A surgical cut guide can be applied to the trial or instrument or patient-specific mold with the spacers optionally interposed between the trial or instrument or patient-specific mold and the cut guide. In this manner, the exact position of the surgical cuts can be influenced and can be adjusted to achieve an optimal result. Someone skilled in the art will recognize other means for optimizing the position of the surgical cuts. For example, expandable or ratchet-like devices can be utilized that can be inserted into the joint or that can be attached or that can touch the trial or instrument or patient-specific mold. Hinge-like mechanisms are applicable. Similarly, jack-like mechanisms are useful. In principal, any mechanical or electrical device useful for fine tuning the position of a cut guide relative to a trial or instrument or patient -specific mold can be used.

A surgeon may desire to influence joint laxity as well as joint alignment. This can be optimized for different flexion and extension, abduction, or adduction, internal and external rotation angles. For this purpose, for example, spacers can be introduced that are attached or that are in contact with one or more trials or instruments or patient-specific molds. The surgeon can intraoperatively evaluate the laxity or tightness of a joint using spacers with different thickness or one or more spacers with the same thickness. For example, spacers can be applied in a knee joint in the presence of one or more instruments or trials or molds and the flexion gap can be evaluated with the knee joint in flexion. Different thickness trials can be used. The terms spacer or insert can be used interchangeably with the term trial.

In certain embodiments, the surgeon can elect to insert different trials or spacers or instruments of different thicknesses in the medial and/or lateral joint space in a knee. This can be done before any bone has been resected, once a first bone resection on a first articular surface has been made and after a second bone resection on a first or second articular surface has been made, such as a femur and a tibia or a medial and a lateral condyle or a medial and a lateral tibia. The joint can be tested for soft-tissue tension, ligament tension, ligament balance and/or flexion or extension gap for different orientations or kinematic requirements using different medial and lateral trial or spacer thicknesses, e.g., at different flexion and extension angles. Surgical bone cuts can subsequently optionally be adapted or changed. Alternatively, different medial and lateral insert thickness or profiles or composite heights can be selected for the tibial component(s). For example, combinations of medial and lateral spacers or trials having thicknesses described in **Table 5** can be inserted.

**Table 5: Exemplary medial and lateral spacer, trial, and/or insert heights that can be used in combination**

| **Medial spacer, trial, and/or insert height** | **Lateral spacer, trial, and/or insert height** | **Note** |
|---|---|---|
| **6 mm-16mm** | 6 mm-16mm | Same medial and lateral spacer, trial, and/or insert height |
| | | |
| **6 mm** | 7 mm-16mm | Different medial and lateral spacer, trial, and/or insert (6 mm medial spacer, trial, and/or insert height with various lateral spacer, trial, and/or insert heights) |
| | | |
| **7 mm** | 6 mm-16mm | Different medial and lateral spacer, trial, and/or insert height (7 mm medial spacer, trial, and/or insert height with various lateral spacer, trial, and/or insert heights) |
| | | |
| **8 mm** | 6 mm-16mm | Different medial and lateral spacer, trial, and/or insert height (8 mm medial spacer, trial, and/or insert height with various lateral spacer, trial, and/or insert heights) |
| **9 mm** | 6 mm-16mm | Different medial and lateral spacer, trial, and/or insert height (9 mm medial spacer, trial, and/or insert height with various lateral spacer, trial, and/or insert heights) |
| **11 mm** | 6 mm-16mm | Different medial and lateral spacer, trial, and/or insert height (11 mm medial spacer, trial, and/or insert height with various lateral spacer, trial, and/or insert heights) |
| **12 mm** | 6 mm-16mm | Different medial and lateral spacer, trial, and/or insert height (12 mm medial spacer, trial, and/or insert height with various lateral spacer, trial, and/or insert heights) |
| **13 mm** | 6 mm-16mm | Different medial and lateral spacer, trial, and/or insert height (13 mm medial spacer, trial, and/or insert height with various lateral spacer, trial, and/or insert heights) |
| **14 mm** | 6 mm-16mm | Different medial and lateral spacer, trial, and/or insert height (14 mm medial spacer, trial, and/or insert height with various lateral spacer, trial, and/or insert heights) |
| **15 mm** | 6 mm-16mm | Different medial and lateral spacer, trial, and/or insert height (15 mm medial spacer, trial, and/or insert height with various lateral spacer, trial, and/or insert heights) |
| **16 mm** | 6 mm-15mm | Different medial and lateral spacer, trial, and/or insert height (16 mm medial spacer, trial, and/or insert height with various lateral spacer, trial, and/or insert heights) |

Thus, by using separate medial and/or lateral spacers or trials or inserts, it is possible to determine an optimized combination of medial or lateral tibial components, for example with regard to medial and lateral composite thickness, insert thickness or medial and lateral implant or insert profile. Thus, medial and/or lateral tibial implant or component or insert thickness can be optimized for a desired soft-tissue or ligament tension or ligament balance for different flexion and extension angles and other joint poses. This offers a unique benefit beyond traditional balancing using bone cuts and soft-tissue releases. In one embodiment, the surgeon can place the tibial and femoral surgical bone cuts and perform the proper soft-tissue or ligament tensioning or balancing entirely via selection of a medial or lateral tibial insert or composite thickness and/or profile. Additional adaptation and optimization of bone cuts and soft-tissue releases is possible.

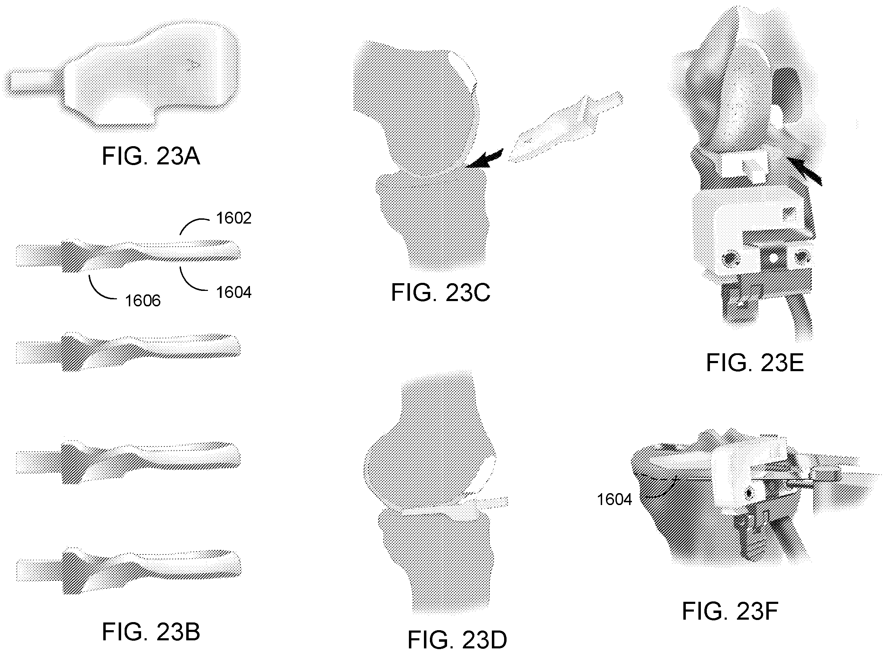

**FIGS. 23A** through **28C** show various exemplary spacers or trials or inserts for adjusting and optimizing alignment, tension, balance, and position during a knee implant surgery. In particular, **FIG. 23A** depicts a medial balancer chip insert from top view to show the superior surface of the chip. **FIG. 23B** depicts a side view of a set of four medial balancer chip inserts that incrementally increase in thickness by 1 mm. A corresponding set of lateral balancing chip inserts (having a range of thicknesses) can be used in conjunction with a set of medial balancing chip inserts. In this way, the joint can be optimized using independent medial and lateral balancing chips inserts having different thicknesses. As indicated with the first chip in the figure, the superior surface **1602** of a balancing chip insert engages the femur and the inferior surface **1604** engages the tibia. In certain embodiments, one or both of the superior surface **1602** and/or the inferior surface **1604** can be patient-adapted to fit the particular patient. In certain embodiments, a balancing chip can include a resection surface to guide a subsequent surgical bone cut.

**FIG. 23C** depicts a medial balancing chip being inserted in flexion between the femur and tibia. **FIG. 23D** depicts the medial balancing chip insert in place while the knee is brought into extension. Optionally, a lateral balancing chip also can be placed between the lateral portions of the femur and tibia. Medial and lateral balancing chips having different thicknesses can be placed as shown in **FIGS. 23C** and **23D****,** until a desired tension is observed medially and laterally throughout the patient's range of motion. As shown in **FIG. 23E****,** in certain embodiments, a cutting guide can be attached to the medial balancing chip insert, to the lateral balancing chip insert, or to both, so that the resection cuts are made based on the selected medial and lateral balancing chip inserts. Optionally, one or more surfaces of one or both balancing chips also can act as a cutting guide. As shown in **FIG. 23F****,** the inferior surface **1604** of the medial balancing chip acts as cutting guide surface for resectioning the medial portion of the tibia.

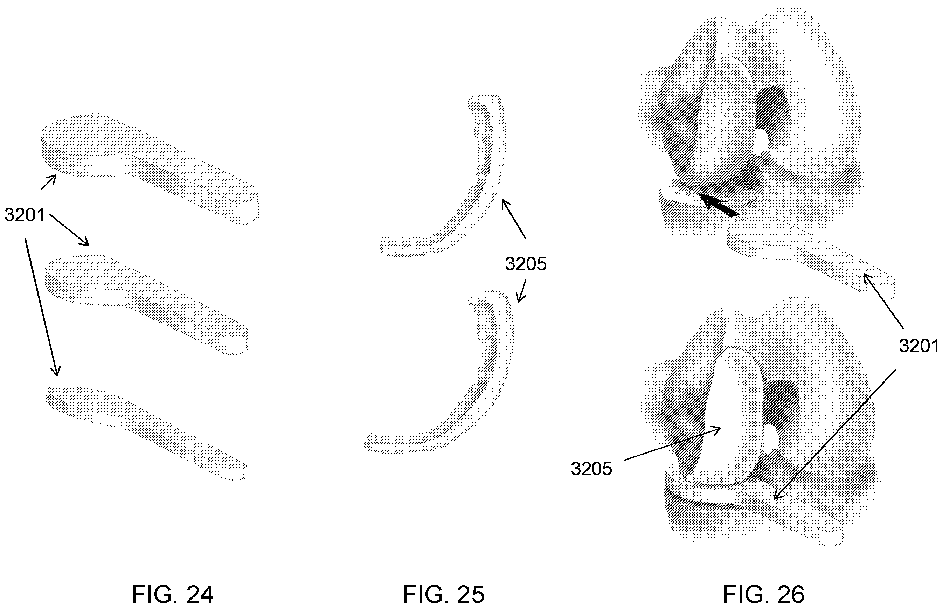

**FIG. 24** depicts a set of three medial spacer block inserts **3201** having incrementally increasing thicknesses, for example, thicknesses that increase by 1 mm, by 1.5 mm, or by 2 mm. A corresponding set of lateral medial spacer block inserts (having a range of thicknesses) can be used in conjunction with a set of medial spacer block inserts. A spacer block insert can be used, for example, to provide the thickness of a tibial implant component (optionally with or without the additional thickness of a tibial implant component insert) during subsequent implantation steps and prior to placement of the tibial implant component. In certain embodiments, the spacer block insert can include a portion for attaching a trial a tibial implant component insert, so that the precise thicknesses of different combinations of tibial implant components and component inserts can be assessed. By using medial and lateral spacer block inserts of different thicknesses, the balancing, tensioning, alignment, and/or positioning of the joint can continue to be optimized throughout the implantation procedure. In certain embodiments, one or more features of a spacer block insert can be patient-adapted to fit the particular patient. In certain embodiments, a spacer block insert can include a feature for attaching or stabilizing a cutting guide and/or a feature for guiding a cutting tool.

**FIG. 25** depicts a set of two medial femoral trials **3205** having incrementally increasing thicknesses, for example, thicknesses that increase by 1 mm, by 1.5 mm, or by 2 mm. A corresponding set of lateral femoral trials (having a range of thicknesses) can be used in conjunction with the set of medial femoral trials. A femoral trial can be used, for example, to test variable thicknesses and/or features of a femoral implant component during implantation steps prior to placement of the tibial implant component. By using medial and lateral femoral trials of different thicknesses, the balancing, tensioning, alignment, and/or positioning of the joint can continue to be optimized throughout the implantation procedure. In certain embodiments, one or more features of a femoral trial can be patient-adapted to fit the particular patient. In certain embodiments, a femoral trial can include a feature for attaching or stabilizing a cutting guide and/or a feature for guiding a cutting tool.

**FIG. 26** depicts a medial femoral trial **3205** in place and a spacer block **3201** being inserted to evaluate the balance of the knee in flexion and extension. Spacer blocks having different thicknesses can be inserted and evaluated until an optimized thickness is identified. Optionally, a lateral femoral trial also can be placed between the lateral portions of the femur and tibia and a lateral spacer block inserted and evaluated along with the medial spacer block. Medial and lateral spacer blocks having different thicknesses can be placed and removed until a desired tension is observed medially and laterally throughout the patient's range of motion. Then, a tibial implant component and/or tibial implant component insert can be selected to have a thickness based on the thickness identified by evaluation using the femoral trial and spacer block. In this way, the selected medial a tibial implant component (and/or tibial implant component insert) and the lateral tibial implant component (and/or tibial implant component insert) can have different thicknesses.

**FIG. 27** depicts a set of three medial tibial component insert trials having incrementally increasing thicknesses, for example, thicknesses that increase by 0.5 mm, by 1 mm, by 1.5 mm, or by 2 mm. A corresponding set of lateral tibial component insert trials (having a range of thicknesses) can be used in conjunction with the set of medial tibial component insert trials. A tibial component insert trial can be used, for example, to determine the best insert thickness and/or features of a tibial component insert during the final implantation steps. By using medial and lateral tibial component insert trials of different thicknesses and/or configurations, the balancing, tensioning, alignment, and/or positioning of the joint can be optimized even in the final steps of the procedure. In certain embodiments, one or more features of a tibial component insert trial can be patient-adapted to fit the particular patient. **FIGS. 28A** and **28B** depict the process of placing and adding various tibial component insert trials and **FIG. 28C** depicts the process of placing the selected tibial component insert.

The sets of exemplary spacers, trials, and inserts described in connection with **FIGS. 23A** through **28C** can be expanded to include spacers, trials, and/or inserts having various intermediate thicknesses (e.g., in increments of 0.5 mm, 0.25 mm, and/or 0.1 mm) and/or having various other selection features. For example, sets of femoral and/or tibial insert trials can include different bone-facing and/or joint-facing surfaces from which the surgeon can select the optimum available surface for further steps in the procedure.

Using the various spacers, trials, and inserts described above, the knee joint can be flexed and the flexion gap can be evaluated. In addition, the knee can be extended and the extension gap can be evaluated. Ultimately, the surgeon will select an optimal combination of spacers or trials for a given joint, instrument, trial or mold. A surgical cut guide can be applied to the trial, instrument, or mold with the spacers optionally interposed between the trial, instrument or mold and the cut guide. In this manner, the exact position of the surgical cuts can be influenced and can be adjusted to achieve an optimal result. Someone skilled in the art will recognize other means for optimizing the position of the surgical cuts. For example, expandable or ratchet-like devices can be utilized that can be inserted into the joint or that can be attached or that can touch the trial, instrument or mold. Hinge-like mechanisms are applicable. Similarly, jack-like mechanisms are useful. In principal, any mechanical or electrical device useful for fine tuning the position of the cut guide relative to the trial or instrument or molds can be used. The trials or instruments or molds and any related instrumentation such as spacers or ratchets can be combined with a tensiometer to provide a better intraoperative assessment of the joint. The tensiometer can be utilized to further optimize the anatomic alignment and tightness or laxity of the joint and to improve postoperative function and outcomes. Optionally local contact pressures may be evaluated intraoperatively, for example using a sensor like the ones manufactured by Tekscan, South Boston, MA.

### EQUIVALENTS

The foregoing embodiments are to be considered in all respects illustrative rather than limiting on the invention described herein. Scope of the invention is thus indicated by the appended claims rather than by the foregoing description.

## Claims

1. A knee implant system, including
a femoral knee implant and
a patient specific tibial knee implant created for a patient, wherein said tibial knee implant comprises:
(a) a tray component for placement on the patient's resected lateral tibial plateau having a perimeter shape that substantially matches the perimeter of the resected surface of the patient's lateral tibial plateau;
(b) a tray component for placement on the patient's resected medial tibial plateau having a perimeter shape that substantially matches the perimeter of the resected surface of the patient's medial tibial plateau;
(c) an insert component for insertion into the tray component for placement on the patient's resected lateral tibial plateau having a joint facing surface for mating with a lateral condyle surface of the femoral implant, wherein the joint facing surface of the insert component substantially negatively matches the curvature of the lateral condyle of the femoral implant; and
(d) an insert component for insertion into the tray component for placement on the patient's resected medial tibial plateau having a joint facing surface for mating with a medial condyle surface of the femoral implant, wherein the joint facing surface of the insert component substantially negatively matches the curvature of the medial condyle of the femoral implant, wherein the lateral condyle or the medial condyle or both have a patient specific sagittal curvature, and
wherein the joint facing surface of the insert component of said tibial knee implant for insertion into the tray component for placement on the patient's resected lateral tibial plateau substantially negatively matches the sagittal curvature, or both the coronal curvature and the sagittal curvature of the lateral condyle surface of a femoral implant, wherein the joint facing surface of the insert component of said tibial knee implant for insertion into the tray component for placement on the patient's resected medial tibial plateau substantially negatively matches the sagittal curvature, or both the coronal curvature and the sagittal curvature of the medial condyle surface of a femoral implant.

2. The knee impant system of claim 1 wherein each of the insert components of said tibial knee implant has the same thickness, or
wherein the insert component for insertion into the tray component for placement on the patient's resected lateral tibial plateau is thicker than the insert component for insertion into the tray component for placement on the patient's resected medial tibial plateau, or
wherein the insert component for insertion into the tray component for placement on the patient's resected medial tibial plateau is thicker than the insert component for insertion into the tray component for placement on the patient's resected lateral tibial plateau.

3. A knee implant system, including
a femoral knee implant and
a patient specific tibial knee implant created for a patient, wherein said tibial knee implant comprises:
(a) a tray component for placement on the patient's resected tibial plateau, wherein the tray component has a medial side and a lateral side and the perimeter shape of the tray component substantially matches the perimeter of the patient's resected tibial plateau;
(b) an insert component for insertion into the lateral side of the tray component having a joint facing surface for mating with the
lateral condyle surface of the femoral implant, wherein the joint facing surface of the insert component substantially negatively matches the curvature of the lateral condyle of the femoral implant; and
(c) an insert component for insertion into the medial side of the tray component having a joint facing surface for mating with the medial condyle surface of the femoral implant, wherein the joint facing surface of the insert component substantially negatively matches the curvature of the medial condyle of the femoral implant
wherein the lateral condyle or the medial condyle or both have a patient specific sagittal curvature, and
wherein the joint facing surface of the insert component of said tibial knee implant for insertion into the lateral side of the tray component substantially negatively matches the sagittal curvature, or both the coronal curvature and the sagittal curvature of the lateral condyle surface of a femoral implant, or
wherein the joint facing surface of the insert component of said tibial knee implant for insertion into the medial side of the tray component substantially negatively matches the the sagittal curvature, or both the coronal curvature and the sagittal curvature of the medial condyle surface of a femoral implant.

4. The knee impant system of claim 3 wherein each of the insert components of said tibial knee implant has the same thickness, or
wherein the insert component for insertion into the lateral side of the tray component is thicker than the insert component for insertion into the medial side of the tray component, or
wherein the insert component for insertion into the medial side of the tray component is thicker than the insert component for insertion into the lateral side of the tray component.

5. A method of creating a tibial knee implant for a patient, comprising:
creating a model of the patient's knee joint, virtually resecting the tibia of the patient's knee joint model to obtain a resected lateral tibial plateau surface and a resected medial tibial plateau surface,
selecting, adapting or designing a lateral tibial tray component to match with a perimeter of the resected lateral tibial plateau surface, and
selecting, adapting or designing a medial tibial tray component to match with a perimeter of the resected medial tibial plateau surface,
selecting, adapting or designing an insert component for insertion into the lateral tray component and
selecting, adapting or designing an insert component for insertion into the medial tibial tray component,
wherein the insert component has a joint-facing surface that has a sagittal curvature derived from the model of the patient's knee joint.

6. The method of claim 5, comprising:
wherein the insert component for insertion into the lateral tibial tray component is thicker than the insert component for insertion into the medial tibial tray component, or
wherein the insert component for insertion into the medial tibial tray component is thicker than the insert component for insertion into the lateral tibial tray component.

7. The method of claim 6, wherein the lateral tibial tray component and the medial tibial tray component are of a unitary device.

8. The method of claim 7, wherein the insert component for insertion into the lateral tibial tray component and the insert component for insertion into the medial tibial tray component are separate devices.

## Patentansprüche

1. Ein Knieimplantatsystem, umfassend
ein femorales Knieimplantat und
ein patientenspezifisches tibiales Knieimplantat, das für einen Patienten entworfen wurde, wobei jenes tibiale Knieimplantat umfasst:
(a) eine Schalenkomponente zum Anordnen auf das resezierte laterale Tibiaplateau des Patienten, das eine Umfangsform aufweist, die im Wesentlichen dem Umfang der resezierten Oberfläche des lateralen Tibiaplateaus des Patienten entspricht;
(b) eine Schalenkomponente zum Anordnen auf das resezierte mediale Tibiaplateau des Patienten, das eine Umfangsform aufweist, die im Wesentlichen dem Umfang der resezierten Oberfläche des medialen Tibiaplateaus des Patienten entspricht;
(c) eine Einsatzkomponente zum Einsetzen in die Schalenkomponente zum Anordnen auf das resezierte laterale Tibiaplateau des Patienten, das eine dem Gelenk zugewandten Oberfläche zum Paaren mit einer lateralen Kondylusoberfläche des Femurimplantats aufweist, wobei die dem Gelenk zugewandte Oberfläche der Einsatzkomponente im Wesentlichen negativ der Krümmung des lateralen Kondylus des Femurimplantats entspricht; und
(d) eine Einsatzkomponente zum Einsetzen in die Schalenkomponente zum Anordnen auf das resezierte mediale Tibiaplateau des Patienten, das eine dem Gelenk zugewandten Oberfläche zum Paaren mit einer medialen Kondylusoberfläche des Femurimplantats aufweist, wobei die dem Gelenk zugewandte Oberfläche der Einsatzkomponente im Wesentlichen negativ der Krümmung des medialen Kondylus des Femurimplantats entspricht,
wobei der laterale Kondylus oder der mediale Kondylus oder beide eine patientenspezifische sagittale Krümmung aufweisen, und
wobei die dem Gelenk zugewandte Oberfläche der Einsatzkomponente jenes tibialen Knieimplantats zum Einsetzen in die Schalenkomponente zum Anordnen auf dem resezierten lateralen Tibiaplateau des Patienten im Wesentlichen negativ der sagittalen Krümmung oder der koronalen und sagittalen Krümmung der lateralen Kondylusoberfläche eines Femurimplantats entspricht,
wobei die dem Gelenk zugewandte Oberfläche der Einsatzkomponente jenes tibialen Knieimplantats zum Einsetzen in die Schalenkomponente zum Anordnen auf dem resezierten medialen Tibiaplateaus des Patienten im Wesentlichen negativ der sagittalen Krümmung oder der koronalen und sagittalen Krümmung der medialen Kondylusoberfläche eines Femurimplantats entspricht.

2. Das Knieimplantatsystem von Anspruch 1, wobei jede der Einsatzkomponenten jenes tibialen Knieimplantatsystems die gleiche Dicke aufweist, oder
wobei die Einsatzkomponente zum Einsetzen in die Schalenkomponente zum Anordnen auf dem resezierten lateralen Tibiaplateaus des Patienten dicker ist als die Einsatzkomponente zum Einsetzen in die Schalenkomponente zum Anordnen auf dem resezierten medialen Tibiaplateaus des Patienten, oder
wobei die Einsatzkomponente zum Einsetzen in die Schalenkomponente zum Anordnen auf dem resezierten medialen Tibiaplateaus des Patienten dicker ist als die Einsatzkomponente zum Einsetzen in die Schalenkomponente zum Anordnen auf dem resezierten lateralen Tibiaplateau des Patienten.

3. Ein Knieimplantatsystem, umfassend
ein femorales Knieimplantat und
ein patientenspezifisches tibiales Knieimplantat, das für einen Patienten entworfen wurde, wobei jenes tibiale Knieimplantat umfasst:
(a) eine Schalenkomponente zum Anordnen auf das resezierte laterale Tibiaplateau des Patienten, wobei die Schalenkomponente eine mediale Seite und eine laterale Seite aufweist und die Umfangsform der Schalenkomponente im Wesentlichen dem Umfang des resezierten Tibiaplateaus des Patienten entspricht;
(b) eine Einsatzkomponente zum Einsetzen in die laterale Seite der Schalenkomponente, die eine dem Gelenk zugewandten Oberfläche zum Paaren mit der lateralen Kondylusoberfläche des Femurimplantats aufweist,
wobei die dem Gelenk zugewandte Oberfläche der Einsatzkomponente im Wesentlichen negativ der Krümmung des lateralen Kondylus des Femurimplantats entspricht; und
(c) eine Einsatzkomponente zum Einsetzen in die mediale Seite der Schalenkomponente, die eine dem Gelenk zugewandten Oberfläche zum Paaren mit der medialen Kondylusoberfläche des Femurimplantats aufweist,
wobei die dem Gelenk zugewandte Oberfläche der Einsatzkomponente im Wesentlichen negativ der Krümmung des medialen Kondylus des Femurimplantats entspricht,
wobei der laterale Kondylus oder der mediale Kondylus oder beide eine patientenspezifische sagittale Krümmung aufweisen, und
wobei die dem Gelenk zugewandte Oberfläche der Einsatzkomponente jenes tibialen Knieimplantats zum Einsetzen in die laterale Seite der Schalenkomponente im Wesentlichen negativ der sagittalen Krümmung oder der koronalen und sagittalen Krümmung der lateralen Kondylusoberfläche eines Femurimplantats entspricht, oder wobei die dem Gelenk zugewandte Oberfläche der Einsatzkomponente jenes tibialen Knieimplantats zum Einsetzen in die mediale Seite der Schalenkomponente im Wesentlichen negativ der sagittalen Krümmung oder der koronalen und sagittalen Krümmung der medialen Kondylusoberfläche eines Femurimplantats entspricht.

4. Das Knieimplantatsystem von Anspruch 3, wobei jede der Einsatzkomponenten jenes tibialen Knieimplantats die gleiche Dicke aufweist, oder
wobei die Einsatzkomponente zum Einsetzen in die laterale Seite der Schalenkomponente dicker ist als die Einsatzkomponente zum Einsetzen in die mediale Seite der Schalenkomponente, oder
wobei die Einsatzkomponente zum Einsetzen in die mediale Seite der Schalenkomponente dicker ist als die Einsatzkomponente zum Einsetzen in die laterale Seite der Schalenkomponente.

5. Ein Verfahren zum Entwerfen eines tibialen Knieimplantats für einen Patienten, umfassend:
Entwerfen eines Models des Kniegelenks des Patienten,
Virtuelles Resezieren der Tibia des Kniegelenkmodels des Patienten, um eine resezierte laterale Tibiaplateauoberfläche und eine resezierte mediale Tibiaplateauoberfläche zu erhalten,
Auswählen, Anpassen oder Gestalten einer lateralen Tibia-Schalenkomponente, die einem Umfang der resezierten lateralen Tibiaplateauoberfläche entspricht, und
Auswählen, Anpassen oder Gestalten einer medialen Tibia-Schalenkomponente, die einem Umfang der resezierten medialen Tibiaplateauoberfläche entspricht,
Auswählen, Anpassen oder Gestalten einer Einsatzkomponente zum Einsetzen in die laterale Schalenkomponente, und
Auswählen, Anpassen oder Gestalten einer Einsatzkomponente zum Einsetzen in die mediale Tibia-Schalenkomponente,
wobei die Einsatzkomponente eine dem Gelenk zugewandte Oberfläche hat, die eine von dem Kniegelenkmodel des Patienten abgeleitete sagittale Krümmung aufweist.

6. Das Verfahren von Anspruch 5, umfassend:
wobei die Einsatzkomponente zum Einsetzen in die laterale Tibia-Schalenkomponente dicker ist als die Einsatzkomponente zum Einsetzen in die mediale Tibia-Schalenkomponente, oder
wobei die Einsatzkomponente zum Einsetzen in die mediale Tibia-Schalenkomponente dicker ist als die Einsatzkomponente zum Einsetzen in die laterale Tibia-Schalenkomponente.

7. Das Verfahren von Anspruch 6, wobei die laterale Tibia-Schalenkomponente und die mediale Tibia-Schalenkomponente eine einheitliche Vorrichtung sind.

8. Das Verfahren von Anspruch 7, wobei die Einsatzkomponente zum Einsetzen in die laterale Tibia-Schalenkomponente und die Einsatzkomponente zum Einsetzen in die mediale Tibia-Schalenkomponente getrennte Vorrichtungen sind.

## Revendications

1. Système d'implant de genou, comprenant
un implant de genou fémoral et
un implant de genou tibial spécifique d'un patient créé pour un patient, dans lequel ledit implant de genou tibial comprend:
(a) un composant plaque pour le placement sur le plateau tibial latéral réséqué du patient ayant une forme de périmètre qui correspond sensiblement au périmètre de la surface réséquée du plateau tibial latéral du patient;
(b) un composant plaque pour le placement sur le plateau tibial médial réséqué du patient ayant une forme de périmètre qui correspond sensiblement au périmètre de la surface réséquée du plateau tibial médial du patient;
(c) un composant insert pour l'insertion dans le composant plaque pour le placement sur le plateau tibial latéral réséqué du patient ayant une surface orientée vers l'articulation pour s'apparier avec une surface de condyle latéral de l'implant fémoral, dans lequel la surface orientée vers l'articulation du composant insert correspond sensiblement de manière négative à la courbure du condyle latéral de l'implant fémoral; et
(d) un composant insert pour l'insertion dans le composant plaque pour le placement sur le plateau tibial médial réséqué du patient ayant une surface orientée vers l'articulation pour s'apparier avec une surface de condyle médical de l'implant fémoral, dans lequel la surface orientée vers l'articulation du composant insert correspond sensiblement de manière négative à la courbure du condyle médial de l'implant fémoral,
dans lequel le condyle latéral ou le condyle médial ou les deux ont une courbure sagittale spécifique du patient, et
dans lequel la surface orientée vers l'articulation du composant insert dudit implant de genou tibial pour l'insertion dans le composant plaque pour le placement sur le plateau tibial latéral réséqué du patient correspond sensiblement de manière négative à la courbure sagittale, ou à la courbure coronale et à la courbure sagittale de la surface de condyle latéral d'un implant fémoral,
dans lequel la surface orientée vers l'articulation du composant insert dudit implant de genou tibial pour l'insertion dans le composant plaque pour le placement sur le plateau tibial médial réséqué du patient correspond sensiblement de manière négative à la courbure sagittale, ou à la courbure coronale et à la courbure sagittale de la surface de condyle médial d'un implant fémoral.

2. Système d'implant de genou selon la revendication 1, dans lequel chacun des composants insert dudit implant de genou tibial a la même épaisseur, ou
dans lequel le composant insert pour l'insertion dans le composant plaque pour le placement sur le plateau tibial latéral réséqué du patient est plus épais que le composant insert pour l'insertion dans le composant plaque pour le placement sur le plateau tibial médial réséqué du patient, ou
dans lequel le composant insert pour l'insertion dans le composant plaque pour le placement sur le plateau tibial médial réséqué du patient est plus épais que le composant insert pour l'insertion dans le composant plaque pour le placement sur le plateau tibial latéral réséqué du patient.

3. Système d'implant de genou, comprenant
un implant de genou fémoral et
un implant de genou tibial spécifique du patient créé pour un patient, dans lequel ledit implant de genou tibial comprend:
(a) un composant plaque pour le placement sur le plateau tibial réséqué du patient, dans lequel le composant plaque a un côté médial et un côté latéral et la forme du périmètre du composant plaque correspond sensiblement au périmètre du plateau tibial réséqué du patient;
(b) un composant insert pour l'insertion dans le côté latéral du composant plaque ayant une surface orientée vers l'articulation pour s'apparier à la surface de condyle latéral de l'implant fémoral, dans lequel la surface orientée vers l'articulation du composant insert correspond sensiblement de manière négative à la courbure du condyle latéral de l'implant fémoral; et
(c) un composant insert pour l'insertion dans le côté médial du composant plaque ayant une surface orientée vers l'articulation pour s'apparier avec la surface de condyle médial de l'implant fémoral, dans lequel la surface orientée vers l'articulation du composant insert correspond sensiblement de manière négative à la courbure du condyle médial de l'implant fémoral;
dans lequel le condyle latéral ou le condyle médial ou les deux ont une courbure sagittale spécifique du patient, et
dans lequel la surface orientée vers l'articulation du composant insert dudit implant de genou tibial pour l'insertion dans le côté latéral du composant plaque correspond sensiblement de manière négative à la courbure sagittale, ou à la courbure coronale et à la courbure sagittale de la surface de condyle latéral d'un implant fémoral, ou
dans lequel la surface orientée vers l'articulation du composant insert dudit implant de genou tibial pour l'insertion dans le côté médial du composant plaque correspond sensiblement de manière négative à la courbure sagittale, ou à la courbure coronale et à la courbure sagittale de la surface de condyle médial d'un implant fémoral.

4. Système d'implant de genou selon la revendication 3, dans lequel chacun des composants insert dudit implant de genou tibial a la même épaisseur, ou
dans lequel le composant insert pour l'insertion dans le côté latéral du composant plaque est plus épais que le composant insert pour l'insertion dans le côté médial du composant plaque, ou
dans lequel le composant insert pour insertion dans le côté médial du composant plaque est plus épais que le composant insert pour l'insertion dans le côté latéral du composant plaque.

5. Procédé de création d'un implant de genou tibial pour un patient, comprenant:
la création d'un modèle de l'articulation de genou du patient,
la résection virtuelle du tibia du modèle d'articulation de genou du patient pour obtenir une surface de plateau tibial latéral réséqué et une surface de plateau tibial médial réséqué,
la sélection, l'adaptation ou la conception d'un composant plaque tibial latéral pour correspondre à un périmètre de la surface de plateau tibial latéral réséqué, et
la sélection, l'adaptation ou la conception d'un composant plaque tibial médial pour correspondre à un périmètre de la surface de plateau tibial médial réséqué,
la sélection, l'adaptation ou la conception d'un composant insert pour l'insertion dans le composant plaque latéral et
la sélection, l'adaptation ou la conception d'un composant insert pour l'insertion dans le composant plaque tibial médial,
dans lequel le composant insert a une surface orientée vers l'articulation qui a une courbure sagittale dérivée du modèle d'articulation de genou du patient.

6. Procédé selon la revendication 5, comprenant:
dans lequel le composant insert pour l'insertion dans le composant plaque tibial latéral est plus épais que le composant insert pour l'insertion dans le composant plaque tibial médial, ou
dans lequel le composant insert pour l'insertion dans le composant plaque tibial médial est plus épais que le composant insert pour l'insertion dans le composant plaque tibial latéral.

7. Procédé selon la revendication 6,
dans lequel le composant plaque tibial latéral et le composant plaque tibial médial font partie d'un dispositif unitaire.

8. Procédé selon la revendication 7,
dans lequel le composant insert pour l'insertion dans le composant plaque tibial latéral et le composant insert pour l'insertion dans le composant plaque tibial médial sont des dispositifs séparés.
